# EUROPEAN PATENT SPECIFICATION

(11) **EP 2 709 991 B1**
(45) Date of publication and mention of the grant of the patent: **02.09.2020**
(21) Application number: 12786739.8
(22) Date of filing: 15.05.2012
(51) Int. Cl.: A61K 31/395, A61P 7/06, C07D 255/02, A61K 45/06, A61K 38/19

(54) **Use of cxcr4 antagonists for treating WHIM syndrome, myelokathexis, neutropenia and lymphocytopenia**
Verwendung von cxcr4-Antagonisten zur Behandlung von WHIM Syndrom, Myelokathexis, Neutropenia und Lymphozytopenia
Utilisation d'antagonistes du cxcr4 pour traiter: syndrome WHIM, myelokathexis, neutropénie et lymphocytopénie

(30) Priority: 16.05.2011 US 201161486632 P
(43) Date of publication of application: 26.03.2014
(73) Proprietor: Genzyme Corporation, Cambridge, MA 02142 (US); University of Washington, Seattle, WA 98105 (US)
(72) Inventor: DALE, David, C., Seattle, WA 98105 (US); BRIDGER, Gary, J., Bellingham, WA 98225 (US); HSU, Frank, J., Cambridge, MA 02142 (US)
(74) Representative: J A Kemp LLP
(86) International application number: PCT/US2012/037970
(87) International publication number: WO 2012/158707

(56) References cited:
- US-A1- 2008 234 294
- US-A1- 2009 221 683
- US-B1- 6 365 583
- BADOLATO ET AL: "Mechanisms of WHIM syndrome", DRUG DISCOVERY TODAY: DISEASE MECHANISMS, ELSEVIER, AMSTERDAM, NL, vol. 2, no. 4, 1 January 2005 (2005-01-01) , pages 479-485, XP005213116, ISSN: 1740-6765, DOI: 10.1016/J.DDMEC.2005.11.010
- MARCO A C NEVES ET AL: "Ligand-guided optimization of CXCR4 homology models for virtual screening using a multiple chemotype approach", JOURNAL OF COMPUTER-AIDED MOLECULAR DESIGN, KLUWER ACADEMIC PUBLISHERS, DO, vol. 24, no. 12, 20 October 2010 (2010-10-20), pages 1023-1033, XP019863448, ISSN: 1573-4951, DOI: 10.1007/S10822-010-9393-X
- LABROSSE B ET AL: "Determinants for sensitivity of human immunodeficiency virus coreceptor CXCR4 to the bicyclam AMD3100", JOURNAL OF VIROLOGY, THE AMERICAN SOCIETY FOR MICROBIOLOGY, US, vol. 72, no. 8, 1 August 1998 (1998-08-01) , pages 6381-6388, XP002224660, ISSN: 0022-538X
- FAITH ET AL.: 'Induction of antibody-mediated neutralization in SIVmac239 by a naturally acquired V3 mutation' VIROLOGY vol. 400, no. 1, 25 April 2010, pages 86 - 92, XP026970709
- SIGNORET ET AL.: 'Differential regulation of CXCR4 and CCR5 endocytosis' JOURNAL OF CELL SCIENCE vol. 111, 1998, pages 2819 - 2830, XP055134651

## Description

The present invention is directed to a certain CXCR4 antagonist for use in treating or preventing WHIM syndrome and certain other diseases or conditions. Corresponding pharmaceutical compositions comprising this and other CXCR4 antagonists are also disclosed.

### BACKGROUND OF THE INVENTION

WHIM (Warts, Hypogammaglobulinemia, Immunodeficiency, Myelokathexis) syndrome is a rare disease characterized by warts, hypogammaglobulinemia, recurrent bacterial infections, and leukopenia and neutropenia. The neutropenia is considered to be attributed to myelokathexis, a form of neutropenia associated with abnormal retention of mature neutrophils in the bone marrow and bone marrow hypercellularity. Balabanian, K., et al, Blood, 105:6, 2449-2457 (2005). WHIM syndrome is a hematological disorder in which mature neutrophils fail to exit the bone marrow. Patients also have variable B- and T-cell deficiencies in which patients exhibit a marked T-cell lymphopenia and may evolve to develop a lymphoproliferative disease and lymphoma.

WHIM syndrome is an autosomal dominant disorder attributable to certain mutations in the CXCR4 gene. Tarzi, M.D., et al, J. Allergy Clin Immunol, 116:5, 1101-1105 (2005); Hernandez, et al. Nat Genet, 34, 70-74 (2003). Human patients suffering from WHIM syndrome typically have a white blood cell (WBC) count that is <1.0 x 10⁹/L accompanied by severe neutropenia and lymphocytopenia. If warts are absent, the condition is usually called "myelokathexis." Zeulzer, W.W., NEngl J Med., 210, 699-670 (1964). Bone marrow examination of such patients generally shows abundant neutrophils with hyper-segmented nuclei and remnants of neutrophils in marrow macrophages. O'Regan S, et al., Blut., 42, 191-196 (1981).
Without being bound by theory, it is believed that certain mutations in CXCR4 presumably prevent the normal release of neutrophils from the bone marrow into the blood. Kawai T, et al, Exp Hematol., 33, 460-468 (2005). While the mechanism for lymphocytopenia is not known, it is believed to be attributable to interruption of the normal trafficking of lymphocytes and their retention in the marrow and other lymphoid tissues. Ma, Q., et al.. Immunity, 10, 463-471 (1999).

Blood cells play a crucial part in maintaining the health and viability of animals, including humans. White blood cells include neutrophils, macrophage, eosinophils and basophils/mast cells as well as the B- and T-cells of the immune system. White blood cells are continuously replaced via the hematopoietic system, by action of colony stimulating factors (CSF) and various cytokines on stem cells and progenitor cells in hematopoietic tissues. Recent data support a model in which myelokathexis may result from impaired neutrophil migration from bone marrow.

CXCR4 is a chemokine receptor G protein coupled receptor (GPCR) that is expressed in a variety of normal tissues, including leukocytes. SDF-1 (CXCL12) is the cognate ligand of this receptor, and is known to act as a chemoattractant that drives chemotaxis of cells expressing CXCR4. Certain mutations in the CXCR4 gene have been associated with WF1IM syndrome and myelokathexis. Furthermore functional alterations of CXCR4-medited responses constitute a common biological trait of this pathology.

We hypothesized that certain CXCR4 antagonists might be useful as a molecularly targeted therapy for myelokathexis or WHIM syndrome, increasing circulating leukocytes by overcoming the excessive binding of the mutant CXCR4 to CXCL12.

We determined that certain CXCR4 antagonists disclosed herein are useful for treating or preventing WHIM syndrome and certain other diseases or conditions disclosed herein (e.g., treating, preventing, or limiting HPV infection).

AnorMED Corporation, now Genzyme Corporation, disclosed certain compounds useful in the present disclosure, which are antagonists of the CXCR4 receptor that prevent its interaction with the cytokine stromal cell derived factor-1 (SDF-1), which is now designated as CXCL12. Many such agents and uses of such agents are known in the art. One notable agent, 1,1'-[1,4-phenylene-bis-(methylene)-bis-1,4,8,11-tetraazacyclotetradecane (also known by its codename,

AMDS 100), is the active ingredient of MOZOBIL® (plerixafor). Plerixafor is a small molecule inhibitor of the binding of CXCR4 to its natural ligand, the chemokine SDF-1, also called CXCL12. Haste S, et al, FEES Lett., 527, 255-262 (2002). Subcutaneous administration of plerixafor causes dose-dependent leukocytosis and increases circulating CD34⁺ cells. Liles W.C., et al.. Blood, 102, 2728-2730 (2003); DiPersio J.F., et al, Blood, 113, 5720-5726 (2009). The FDA recently granted Genzyme Corporation approval to market Mozobil for use in combination with granulocyte-colony stimulating factor (G-CSF) to mobilize hematopoietic stem cells to the peripheral blood for collection and subsequent autologous transplantation in patients with non-Hodgkin's lymphoma (NHL) and multiple myeloma (MM). This CXCR4 antagonist and other CXCR4 antagonists are disclosed, for example, in U.S. Patent Nos. 5,021,409; 6,001,826; 5,583,131; RE42,152; 5,698,546; 5,817,807; 6,756,391; 7,022,717; 7,160,872; 7,414,065; 7,7094,86; 6,506,770; 6,667,320; 6,872,714; in U.S. Patent Application Publication No. 2007/0060591; and in WO 92/016494; WO 93/012096; WO 95/018808; WO 00/002870 and WO 01/044229.

In addition, we have previously disclosed improved methods for preparing certain of the presently disclosed CXCR4 antagonists, including plerixafor, in U.S. Patent Nos. 5,612,478; 5,756,728; 5,801,281; and 5,606,053; 6,489,472 and WO 02/26721.

We have discovered, and have disclosed in WO 00/045814, that certain CXCR4 antagonists, such as plerixafor, have the effect of increasing the white blood cell count. We have also found, and have disclosed in U.S. 6,987,102; 7,897,590; 7,935,692; and WO 03/011277, that these antagonists have the effect of mobilizing progenitor cells and/or stem cells from the bone marrow to the circulating blood for use in part in hematopoietic stem cell transplants. Certain uses of CXCR4 antagonists are disclosed in U.S. Patent Application Publication Nos. 2007/0043012;
2010/0035941; and WO 06/020891; WO 08/017025; WO 08/019371; WO 2010/025416; WO 2010/088398; and WO 2010/08840.

None of the foregoing documents specifically mention the use of a CXCR4 antagonist disclosed therein for the treatment or prevention of WHIM syndrome or myelokathexis or the diseases or conditions disclosed herein. As disclosed herein, we have unexpectedly discovered that the CXCR4 antagonists disclosed herein are active for the treatment or prevention of WHIM syndrome and certain other diseases or conditions - a significant unmet medical need.
Badolato et al, Drug Discov. Today Dis. Mech, 2, 479-485 (2005) describes drugs that inhibit CXCL12 binding to CXCR4; Nevas, Marco A. C, et al. J Comput. Aided Mol. Des., 24, 1023-1033 (2010) describes ligand-receptor models of CXCR4 useful for elucidation of the molecular determinants of small molecule binding and functional antagonism.

### SUMMARY OF THE INVENTION

Disclosed herein is the treatment or prevention of WHIM syndrome and certain other diseases or conditions. While these methods are relevant for use in a clinical setting for a patient having WHIM syndrome and these other disorders or conditions, it is envisioned that such a patient will be able to perform the methods need not be carried out in a hospital setting. For example, such patients may be able to practice the presently disclosed methods at home.

The present invention provides a compound for use in a method of:
- treating or preventing WHIM syndrome in a human;
- treating or preventing myelokathexis in a human; or
- correcting neutropenia and/or lymphocytopenia in a human patient with myelokathexis or WHIM syndrome;
wherein the compound is a CXCR4 antagonist that is 1,1'-[1,4-phenylene-bis-(methylene)]-bis-1,4,8,11-tetraazacyclotetradecane or a pharmaceutically acceptable salt or metal complex thereof.

. The scope of the invention is defined by the claims. Any references in the description to methods of treatment refer to the compounds, pharmaceutical compositions and medicaments of the present invention for use in a method for treatment of the human (or animal) body by therapy.

In another aspect, the present invention is directed to a compound for use as defined above, wherein the method comprises testing for the presence of a CXCR4 mutation in a human having myelokathexis or WHIM syndrome; selecting a human having the CXCR4 mutation; and administering to the selected human a CXCR4 antagonist as defined above in an amount effective to treat or prevent myelokathexis or WHIM syndrome.

In another aspect, the present invention is directed to a compound for use as defined above, wherein the compound is in the form of an acid addition salt.

In another aspect, the present invention is directed to a compound for use as defined above, wherein the acid addition salt is hydrochloride.

In another aspect, the present invention is directed to a compound for use as defined above, wherein the human has the CXCR4 mutation R334.

In another aspect, the present invention is directed to a compound for use as defined above, wherein the human has the CXCR4 mutation S324fs365.

In another aspect, the present invention is directed to a compound for use as defined above, wherein the compound is administered at a dose range of between about 0.04 mg/kg to 0.24 mg/kg.

In another aspect, the present invention is directed to a compound for use as defined above, wherein the compound is administered to the human by a subcutaneous route. In another aspect, the present invention is directed to a compound for use as defined above, wherein the compound is administered in combination with G-CSF. Disclosed herein is a method for treating or preventing WHIM syndrome in a human in need thereof comprising administering to said human a CXCR4 antagonist in an amount effective to treat or prevent the WHIM syndrome, wherein the CXCR4 antagonist is a compound of formula I:

Z-linker-Z' (I)

or a pharmaceutically acceptable salt or metal complex thereof, wherein Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

-N(R)-(CR₂)n-X

wherein each R is independently H or straight, branched or cyclic alkyl (1- 6C), n is 1 or 2, and X is an aromatic ring, including heteroaromatic rings, or is a mercaptan,
or Z' is of the formula

-Ar(Y)j

wherein Ar is an aromatic or heteroaromatic moiety, and each Y is independently a non-interfering substituent and j is 0-3; and
"linker" represents a bond, alkylene (1-6C) or may comprise aryl, fused aryl, oxygen atoms contained in an alkylene chain, or may contain keto groups or nitrogen or sulfur atoms.

In certain embodiments in the compounds of formula I, Z and Z' are identical. In certain of these embodiments, Z and Z' are both cyclic polyamines, where in certain embodiments the cyclic polyamine contains 10-24 members and contains 4 nitrogen atoms. In certain of these embodiments, Z and Z' are both 1,4,8,11-tetraazacyclotetradecane. In certain embodiments, the linker is an aromatic ring bracketed by two methylene moieties. In certain of these embodiments, the linker is 1,4-phenylene-bismethylene. A particularly preferred CXCR4 antagonist of formula I is plerixafor (i.e., 1,1'-[1,4-phenylene-bis-(methylene)]-bis-1,4,8,11-tetraazacyclotetradecane) or a pharmaceutically acceptable salt or metal complex thereof. Plerixafor has been found to have the ability of increasing circulating CD34⁺ cells.

In certain embodiments in the compounds of formula I, Z¹ is of the formula

-N(R)-(CR₂)n-X

wherein each R is independently H or straight, branched or cyclic alkyl (1-6C), n is 1 or 2, and X is an aromatic ring, including heteroaromatic rings, or is a mercaptan. In certain embodiments, each R is H, n is 2 and X is substituted or unsubstituted pyridyl. In certain of these embodiments, Z' is 2aminomethyl-pyridine. A compound within these embodiments is N-[1,4,8,11-tetraazacyclotetradecanyl(1,4-phenylene-bis-(methylene)]-2-aminoethyl-2-pyridine or a pharmaceutically acceptable salt or metal complex thereof.

In certain embodiments in the compounds of formula I, the compound is selected from:
3,3'-bis-1,5,9,13-tetraazacyclohexadecane;
3,3'-bis-1,5,8,11,14-pentaazacyclohexadecane;
5,5'-bis-1,4,8,11-tetraazacyclotetradecane;
2,5'-bis-1,4,8,11-tetraazacyclotetradecane;
2,6'-bis-1,4,8,11-tetraazacyclotetradecane;
methylene (or polymethylene) di 1-N-1,4,8,11-tetraazacyclotetradecane;
11,11'-(1,2-ethanediyl)bis-1,4,8,11-tetraazacyclotetradecane;
11,11'-(1,2-propanediyl)bis-1,4,8,11-tetraazacyclotetradecane;
11,11'-(1,2-butanediyl)bis-1,4,8,11-tetraazacyclotetradecane;
11.11'-(1,2-pentanediyl)bis-1,4,8,11-tetraazacyclotetradecane;
11.11'-(1,2-hexanediyl)bis-1,4,8,11-tetraazacyclotetradecane;
1.1'-[1,3-phenylene-bis(methylene)]-bis-1,4,8,11-tetraazacyclotetradecane;
1,1'-[1,4-phenylene-bis(methylene)]-bis-1,4,8,11-tetraazacyclotetradecane;
1,1'-[3,3'-biphenylene-bis-(methylene)]-bis-1,4,8,11-tetraazacyclotetradecane;
11.11'-[1,4-phenylene-bis-(methylene)]-bis-1,4,7,11-tetraazacyclotetradecane;
1.11'-[1,4-phenylene-bis(methylene)]-1,4,8,11-tetraazacyclotetradecane;
1.1'-[2,6-pyridine-bis-(methylene)]-bis-1,4,8,11-tetraazacyclotetradecane;
1,1-[3,5-pyridine-bis-(methylene)]-bis-1,4,8,11-tetraazacyclotetradecane;
1,1'-[2,5-thiophene-bis-(methylene)]-bis-1,4,8,11-tetraazacyclotetradecane;
1,1'-[4,4'-(2,2'-bipyridine)-bis-(methylene)] -bis-1,4,8,11-tetraazacyclotetradecane;
1,1'-[2,9-(1,10-phenanthroline)-bis-(methylene)]-bis-1,4,8,11-tetraazacyclotetradecane;
1,1'-[1,3-phenylene-bis-(methylene)]-bis-1,4,7,10-tetraazacyclotetradecane;
1,1'-[1,4-phenylene-bis-(methylene)]-bis-1,4,7,10-tetraazacyclotetradecane;
1,1'-[5-nitro-1,3-phenylenebis(methylene)]bis-1,4,8,11-tetraazacyclotetradecane;
1,1'-[2,4,5,6-tetrachloro-1,3-phenyleneis(methylene)]bis-1,4,8,11 - tetraazacyclotetradecane;
1,1'-[2,3,5,6-tetrafluoro-1,4-phenylenebis(methylene)]bis-1,4,8,11-tetraazacyclotetradecane;
1,1'-[1,4-naphthylene-bis-(methylene)]bis-1,4,8,11-tetraazacyclotetradecane;
1,1'-[1,3-phenylenebis-(methylene)]bis-1,5,9-triazacyclododecane;
1,1'-[1,4-phenylene-bis-(methylene)]-1,5,9-triazacyclododecane;
1,1'-[2,5-dimethyl-1,4-phenylenebis-(methylene)]-bis-1,4,8,11-tetraazacyclotetradecane;
1,1'-[2,5-dichloro-1,4-phenylenebis-(methylene)]-bis-1,4,8,11-tetraazacyclotetradecane;
1,1'-[2-bromo-1,4-phenylenebis-(methylene)]-bis-1,4,8,11-tetraazacyclotetradecane;
1,1'-[6-phenyl-2,4-pyridinebis-(methylene)]-bis-1,4,8,11-tetraazacyclotetradecane;
7,7'-[14-phenylene-bis(methylene)]bis-3,7,11,17-tetraazabicyclo[13.3.1]heptadeca-1 (17), 13,15 triene;
7,7'-[1,4-phenylene-bis(methylene)]bis[15-chloro-3,7,11,17-tetraazabicyclo [13.3.1]heptadecal(17),13,15-triene];
7,7'-[1,4-phenylene-bis(methylene)]bis[15-methoxy-3,7,11,17-tetraazabicyclo[13.3.1]heptadeca 1(17),13,15 -triene];
7,7'-[1,4-phenylene-bis(methylene)]bis-3,7,11,17-tetraazabicyclo[13.3.1]-heptadeca-13,16-triene 15-one;
7,7'-[1,4-phenylene-bis(methylene)]bis-4,7,10,17-tetraazabicyclo[13.3.1]-heptadeca-1 (17), 13,15-triene;
8,8'-[1,4-phenylene-bis(methylene)]bis-4,8,12,19-tetraazabicyclo[15.3.1]nonadeca-1 (19), 15,17triene;
6,6'-[1,4-phenylene-bis(methylene)]bis-3,6,9,15-tetraazabicyclo[11.3.1]pentadeca-1(15),11,13triene;
6,6'-[1,3-phenylene-bis(methylene)]bis-3,6,9,15-tetraazabicyclo[11.3.1]pentadeca-1(15),11,13triene;
17,17'-[1,4-phenylene-bis(methylene)]bis-3,6,14,17,23,24-hexaazatricyclo[17.3.1.18,12]tetracosa1(23),8,10,12(24),19,21-hexaene;
N-[1,4,8,11-tetraazacyclotetradecanyl-1,4-phenylenebis(methylene)]-2-(amino-methyl)pyridine ;
N-[1,4,8,11-tetraazacyclotetradecanyl-1,4-phenylenebis(methylene)]-N-methyl-2(aminomethyl)pyridine;
N-[1,4,8,11-tetraazacyclotetradecanyl-1,4-phenylenebis(methylene)]-4-(amino-methyl)pyridine;
N-[1,4,8,11-tetraazacyclotetradecanyl-1,4-phenylenebis(methylene)]-3-(amino-methyl)pyridine;
N-[1,4,8,11-tetraazacyclotetradecanyl-1,4-phenylenebis(methylene)]-(2-amino-methyl-5methyl)pyrazine;
N-[1,4,8,11-tetraazacyclotetradecanyl-1,4-phenylenebis(methylene)]-2-(amino-ethyl)pyridine;
N-[1,4,8,11-tetraazacyclotetradecanyl-1,4-phenylenebis(methylene)]-2-(amino-methyl)thiophene;
N-[1,4,8,11-tetraazacyclotetradecanyl-1,4-phenylenebis(methylene)]-2-(amino-ethyl)mercaptan;
N-[1,4,8,11-tetraazacyclotetradecanyl-1,4-phenylenebis(methylene)]-2-amino-benzylamine;
N-[1,4,8,11-tetraazacyclotetradecanyl-1,4-phenylenebis(methylene)]-4-amino-benzylamine;
N-[1,4,8,11-tetraazacyclotetradecanyl-1,4-phenylenebis(methylene)]-4-(amino-ethyl)imidazole;
N-[1,4,8,11-tetraazacyclotetradecanyl-1,4-phenylenebis(methylene)]-benzylamine;
N-[1,4,8,11-tetraazacyclotetradecanyl-1,4-phenylenebis(methylene)]-purine;
N-[1,4,8,11-tetraazacyclotetradecanyl-1,4-phenylenebis(methylene)]-4-phenylpiperazine;
1-[2,6-dimethoxypyrid-4-yl(methylene)]-1,4,8,11-tetraazacyclotetradecane;
1-[2-chloropyrid-4-yl (methylene)]-1,4,8,11-tetraazacyclotetradecane;
1-[2,6-dimethylpyrid-4-yl(methylene)]-1,4,8,11-tetraazacyclotetradecane;
1-[2-methylpyrid-4-yl(methylene)]-1,4,8,11-tetraazacyclotetradecane;
1-[2,6-dichloropyrid-4-yl (methylene)]-1,4,8,11-tetraazacyclotetradecane;
1-[2-chloropyrid-5-yl(methylene)]-1,4,8,11-tetraazacyclotetradecane;
7-[4-methylphenyl (methylene)]-4,7,10,17-tetraazabicyclo[13.3.1]heptadeca-1(17),13,15-triene;
N-[4-(1,4,7-triazacyclotetra-decanyl)-1,4-phenylenebis(methylene)]-2-(aminomethyl)pyridine;
N-[1-(1,4,7-triazacyclotetra-decanyl)-1,4-phenylenebis(methylene)]-2-(aminomethyl)pyridine;
N-[7-(4,7,10,17-tetraazabicyclo[13.3.1]heptadeca-1(17),13,15-trienyl)-1,4 phenylenebis(methylene)]-2-(aminomethyl)pyridine;
N-[7-(4,7,10-triazabicyclo[13.3.1]heptadeca-1(17),13,15-trienyl)-1,4-phenylenebis(methylene)]-2(aminomethyl)pyridine;
N-[4-[4,7,10-triazabicyclo[13.3.1]heptadeca-1(17),13,15-trienyl]-1,4-phenylenebis(methylene)]-2(aminomethyl)pyridine;
N-[4-[4,7,10,17-tetraazabicyclo[13.3.1]heptadeca-1(17),13,15-trienyl]-1,4-phenylenebis(methylene)]-2-(aminomethyl)pyridine;
N-[3-(3,6,17-triazabicyclo[13.3.1]heptadeca-1(17),13,15-trienyl)-1,4-phenylenebis(methylene)]-2(aminomethyl)pyridine;
N-[3-(3,6,17-triazabicyclo[13.3.1]heptadeca-1(17),13,15 -trienyl)-1,3-phenylenebis(methylene)]-2(aminomethyl)pyridine;
N-[4-(4,7,17-triazabicyclo[13.3.1]heptadeca-1(17), 13,15-trienyl)-1,4-phenylenebis(methylene)]-2(aminomethyl)pyridine;
N-[7-(4,7,17-triazabicyclo[13.3.1]heptadeca-1(17),13,15-trienyl)-1,4-phenylenebis(methylene)]-2(aminomethyl)pyridine;
N-[6-(3,6,9-triazabicyclo[11.3.1]pentadeca-1(15),11,13-trienyl)-1,3-phenylenebis(methylene)]-2(aminomethyl)pyridine;
N-[4-(1,7-diazacyclotetradecanyl)-1,4-phenylenebis(methylene)]-2-(aminomethyl)pyridine;
N-[7-(4,10-diazabicyclo[13.3.1]heptadeca-1(17),13,15-trienyl)-1,4-phenylenebis(methylene)]-2(aminomethyl)pyridine;
N-[7-(4,10,17-triazabicyclo[13.3.1]heptadeca-1(17),13,15-trienyl)-1,4-phenylenebis(methylene)]2-(aminomethyl)pyridine;
N-[4-(11-fluoro-1,4,7-triazacyclotetradecanyl)-1,4-phenylenebis(methylene)]-2(aminomethyl)pyridine;
N-[4-(11.11-difluoro-1,4,7-triazacyclotetradecanyl)-1,4-phenylenebis(methylene)]-2(aminomethyl)pyridine;
N-[4-(1,4,7-triazacyclotetradecan-2-onyl)-1,4-phenylenebis(methylene)]-2-(aminomethyl)pyridine;
N-[12-(5-oxa-1,9-diazacyclotetradecanyl)-1,4-phenylenebis(methylene)]-2-(aminomethyl)pyridine;
N-[4-(11-oxa-1,4,7-triazacyclotetradecanyl)-1,4-phenylenebis(methylene)]-2(aminomethyl)pyridine;
N-[4-(11-thia-1,4,7-triazacyclotetradecanyl)-1,4-phenylenebis(methylene)]-2(aminomethyl)pyridine;
N-[4-(11-sulfoxo-1,4,7-triazacyclotetradecanyl)-1,4-phenylenebis(methylene)]-2(aminomethyl)pyridine;
N-[4-(11-sulfono-1,4,7-triazacyclotetradecanyl)-1,4-phenylenebis(methylene)]-2(aminomethyl)pyridine; and
N-[4-(3-carboxo-1,4,7-triazacyclotetradecanyl)-1,4-phenylenebis(methylene)]-2(aminomethyl)pyridine,
or a pharmaceutically acceptable salt or metal complex thereof.

Disclosed herein is a pharmaceutical composition for treating or preventing WHIM syndrome or a disease or condition associated with WHIM syndrome in a human in need thereof comprising:
a compound of formula I:

Z-linker-Z' (I)

or a pharmaceutically acceptable salt or metal complex thereof, wherein
Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

   -N(R)-(CR₂)n-X

   wherein each R is independently H or straight, branched or cyclic alkyl (1-6C), n is 1 or 2, and X is an aromatic ring, including heteroaromatic rings, or is a mercaptan,
or Z' is of the formula

   -Ar(Y)j

   wherein Ar is an aromatic or heteroaromatic moiety, and each Y is independently a non-interfering substituent and j is 0-3; and
   "linker" represents a bond, alkylene (1-6C) or may comprise aryl, fused aryl, oxygen atoms contained in an alkylene chain, or may contain keto groups or nitrogen or sulfur atoms; and
   a pharmaceutically acceptable diluent or carrier.

In certain embodiments of the compound of formula I, the CXCR4 antagonist is 1,1'-[1,4-phenylene-bis-(methylene)]-bis-1,4,8,11-tetraazacyclotetradecane or a pharmaceutically acceptable salt or metal complex thereof. In certain other embodiments, the CXCR4 antagonist is N[1,4,8,11-tetraazacyclotetradecanyl-(1,4-phenylene-bis-(methylene)] -2-aminoethyl-2-pyridine or a pharmaceutically acceptable salt or metal complex thereof.

In certain embodiments, the present disclosure provides a compound for use in a method to treat or prevent WHIM syndrome or a disease or condition disclosed herein in a human in need thereof, wherein the method comprises administering to said human an amount of a compound sufficient to treat or prevent WHIM syndrome or a disease or condition disclosed herein, wherein the compound is a CXCR4 antagonist that is a compound of formula I as defined herein. In certain embodiments, the CXCR4 antagonist is plerixafor.

Disclosed herein is a method for treating or preventing a disorder or condition independently selected from hypogammaglobulinemia, myelokathexis, neutropenia, leukopenia, lymphopenia, abnormalities attributed to improper trafficking of leukocytes, SCID's, diphtheria, bacterial infections, viral infections, or reduced immune function in a human in need thereof comprising administering to said human a CXCR4 antagonist in an amount effective to treat or prevent the disorder or condition, wherein the CXCR4 antagonist is a compound of formula I:

Z-linker-Z' (I)

or a pharmaceutically acceptable salt or metal complex thereof, wherein
Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

   -N(R)-(CR₂)n-X

   wherein each R is independently H or straight, branched or cyclic alkyl (1-6C), n is 1 or 2, and X is an aromatic ring, including heteroaromatic rings, or is a mercaptan, or Z' is of the formula

   -Ar(Y)j

   wherein Ar is an aromatic or heteroaromatic moiety, and each Y is independently a non-interfering substituent and j is 0-3; and
   "linker" represents a bond, alkylene (1-6C) or may comprise aryl, fused aryl, oxygen atoms contained in an alkylene chain, or may contain keto groups or nitrogen or sulfur atoms.

In certain embodiments, the CXCR4 antagonist is 1,1'-[1,4-phenylene-bis-(methylene)]-bis-1,4,8,11-tetraazacyclotetradecane or a pharmaceutically acceptable salt or metal complex thereof.

Disclosed herein are pharmaceutical compositions for treating or preventing a disorder or condition independently selected from hypogammaglobulinemia, myelokathexis, neutropenia, leukopenia, lymphopenia, abnormalities attributed to improper trafficking of leukocytes, SCID's, diphtheria, bacterial infections, viral infections, or reduced immune function in a human in need thereof comprising: a compound of formula I:

Z-linker-Z' (I)

or a pharmaceutically acceptable salt or metal complex thereof, wherein
Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

   -N(R)-(CR₂)n-X

   wherein each R is independently H or straight, branched or cyclic alkyl (1-6C), n is 1 or 2, and X is an aromatic ring, including heteroaromatic rings, or is a mercaptan, or Z' is of the formula

   -Ar(Y)j

   wherein Ar is an aromatic or heteroaromatic moiety, and each Y is independently a non-interfering substituent and j is 0-3; and
   "linker" represents a bond, alkylene (1-6C) or may comprise aryl, fused aryl, oxygen atoms contained in an alkylene chain, or may contain keto groups or nitrogen or sulfur atoms; and
   a pharmaceutically acceptable diluents or carrier.

Disclosed herein are methods for treating or preventing a disorder or condition independently selected from WHIM syndrome, hypogammaglobulinemia, myelokathexis, neutropenia, leukopenia, lymphopenia, abnormalities attributed to improper trafficking of leukocytes, SCID's, diphtheria, bacterial infections, viral infections, or reduced immune function in a human in need thereof comprising
testing for the presence of a CXCR4 mutation in a human having one of the disorders or conditions;
selecting a human having the CXCR4 mutation;
administering to the selected human a CXCR4 antagonist in an amount effective to treat or prevent the disorder or condition, wherein the CXCR4 antagonist is a compound of formula I:

Z-linker-Z' (I)

or a pharmaceutically acceptable salt or metal complex thereof, wherein
Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

   -N(R)-(CR₂),,-X

   wherein each R is independently H or straight, branched or cyclic alkyl (1-6C), n is 1 or 2, and X is an aromatic ring, including heteroaromatic rings, or is a mercaptan,
or Z' is of the formula

   -Ar(Y)j

   wherein Ar is an aromatic or heteroaromatic moiety, and each Y is independently a non-interfering substituent and j is 0-3; and
   "linker" represents a bond, alkylene (1-6C) or may comprise aryl, fused aryl, oxygen atoms contained in an alkylene chain, or may contain keto groups or nitrogen or sulfur atoms.

In certain embodiments, the CXCR4 antagonist is 1,1'-[1,4-phenylene-bis-(methylene)]-bis-1,4,8,11-tetraazacyclotetradecane or a pharmaceutically acceptable salt or metal complex thereof.

In certain embodiments, the human patient with myelokathexis or WHIM syndrome has the CXCR4 mutation R334 or S324fs365.

In certain embodiments, the CXCR4 antagonist is 1,1'-[1,4-phenylene-bis-(methylene)-bis-1,4,8,11-tetraazacyclotetradecane or a pharmaceutically acceptable salt or metal complex thereof.

### BRIEF DESCRIPTION OF THE DRAWINGS

FIG. 1 illustrates neutrophil, lymphocyte, and monocyte counts plotted against time for normal and WHIM syndrome affected human patients when administered plerixafor at a dose range between 0.04 mg/kg-0.24 mg/kg.
FIG. 2 illustrates plots of the amount of cells/pL against time for normal and WHIM syndrome affected human patients when administered plerixafor at 0.08 mg/kg, w here the cell types measured are CD34⁺ cells, T cells, B cells, CD4 T cells, NK cells, and CDS T cells.

### DETAILED DESCRIPTION OF THE INVENTION

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted.

### Definitions:

As used herein, the terms "a" or "an" means "at least one" or "one or more." A group of items linked with the conjunction "or" should not be read as requiring mutual exclusivity among that group, but rather should also be read as "and/or" unless expressly stated otherwise.

As used herein, the terms "treatment" or "treating" refers to any manner in which the symptoms of a condition, disorder or disease are ameliorated or otherwise beneficially altered. Full eradication of the condition, disorder or disease is not required. Amelioration of symptoms of a particular disorder refers to any lessening of symptoms, whether permanent or temporary, that can be attributed to or associated with administration of a therapeutic composition of the present invention or the corresponding methods and combination therapies. Treatment also encompasses pharmaceutical use of the compositions in accordance with the methods disclosed herein.

As used herein, the terms "administration" or "administering" refers to any suitable method of providing a compound or composition presently disclosed to a human subject.

As used herein, the term "effective amount" or "amount effective" or "therapeutically effective amount" of a compound or composition refers to a nontoxic but sufficient amount of the compound to provide the desired therapeutic or prophylactic effect to most patients or individuals. The effective amount of a pharmacologically active compound may vary depending on the route of administration, as well as the age, weight, and sex of the individual to which the drug or pharmacologically active agent is administered. Those of skill in the art given the benefit of the present disclosure will be able to determine appropriate effective amounts by taking into account metabolism, bioavailability, and other factors that affect plasma levels of a compound following administration within the unit dose ranges disclosed further herein for different routes of administration.

As used herein, the term "pharmaceutically acceptable salt" means either a pharmaceutically acceptable acid addition salt or a pharmaceutically acceptable base addition salt of a currently disclosed compound that may be administered without any resultant substantial undesirable biological effect(s) or any resultant deleterious interaction(s) with any other component of a pharmaceutical composition in which it may be contained.

As used herein, the term "prodrug" means a pharmacological derivative of a parent drug molecule that requires biotransformation, either spontaneous or enzymatic, within the organism to release the active drug. For example, prodrugs are variations or derivatives of the compounds of Formula I that have groups cleavable under certain metabolic conditions, which when cleaved, become the compounds of Formula I. Such prodrugs then are pharmaceutically active in vivo, when they undergo solvolysis under physiological conditions or undergo enzymatic degradation. Prodrug compounds herein may be called single, double, triple, etc., depending on the number of biotransformation steps required to release the active drug within the organism, and the number of functionalities present in a precursor-type form. Prodrug forms often offer advantages of solubility, tissue compatibility, or delayed release in the mammalian organism (See, Bundgard, Design of Prodrugs, pp. 7-9, 21-24, Elsevier, Amsterdam 1985 and Silverman, The Organic Chemistry of Drug Design and Drug Action, pp. 352-401, Academic Press, San Diego, Calif., 1992). Prodrugs commonly known in the art include well-known acid derivatives, such as, for example, esters prepared by reaction of the parent acids with a suitable alcohol, amides prepared by reaction of the parent acid compound with an amine, basic groups reacted to form an acylated base derivative. Of course, other prodrug derivatives may be combined with other features disclosed herein to enhance bioavailability.

As indicated above, we discovered that the CXCR4 antagonists disclosed herein are potentially useful as active ingredients for the treatment or prevention of the rare disease known as WHIM syndrome. There currently is no approved drug to treat WHIM syndrome and without such a treatment available, a human patient suffering from WHIM syndrome can ultimately die. Accordingly, this is a clinical area of significant unmet medical need.

WHIM syndrome (warts, hypogammaglobulinemia, immunodeficiency and myelokathexis), which is characterized by chronic noncyclic neutropenia, manifests itself in human patients in a variety of ways. For instance, a human subject or patient diagnosed with WHIM syndrome can have warts on the hands and feet to such a degree that the patient can be incapacitated. Patients afflicted with WHIM syndrome can exhibit increased susceptibility to bacterial and viral infections, especially from common serotype HPY, resulting in warts that can begin development in childhood. Myelokathexis refers to the retention of neutrophils in the bone marrow. WHIM syndrome is often characterized by deficient levels of lymphocytes and antibody levels (gammaglobulins).

WHIM syndrome can result from autosomal dominant mutations in the CXCR4 gene that results in a carboxy-terminus truncation of the receptor of between 10 and 19 residues. Hernandez, etal, Nat. Genet., 34(1): 70-74 (2003); Kawai, T., et al. Blood, 109(1): 78-84 (2007); Aprikyan, A.A.G, et al, Blood, 95, 320-327 (2000). The gene mutant is located on 2q21. It is believed that the because of this truncation of the receptor protein, it is unable to downregulate after stimulation, leaving the receptor in an activated state. Lagane, et al.. Blood, 112(1): 34-44 (2008). While not being bound by theory, it is believed that WHIM syndrome is related to the role of cell signaling and trafficking.

### CXCR4 Antagonists as an Active Pharmaceutical Ingredient:

Suitable CXCR4 antagonists for the instantly disclosed methods and compositions include the compounds of formula I:

Z-linker-Z' (I)

or a pharmaceutically acceptable salt or metal complex thereof, wherein Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

-N(R)-(CR₂)n-X

wherein each R is independently H or straight, branched or cyclic alkyl (1-6C), n is 1 or 2, and X is an aromatic ring, including heteroaromatic rings, or is a mercaptan,
or Z' is of the formula

-Ar(Y)j

wherein Ar is an aromatic or hetero aromatic moiety, and each Y is independently a non-interfering substituent and j is 0-3; and
"linker" represents a bond, alkylene (1-6C) or may comprise aryl, fused aryl, oxygen atoms contained in an alkylene chain, or may contain keto groups or nitrogen or sulfur atoms.

Suitable CXCR4 antagonists for the instantly disclosed methods and compositions also include the compounds of formula

Z-linker-Z' (I)

or a pharmaceutically acceptable salt thereof, wherein
Z is a cyclic polyamine containing 9 to 32 ring members of which 2 to 8 are nitrogen atoms, said nitrogen atoms being separate d from each other by at least 2 carbon atoms, and wherein said heterocycle may optionally contain additional heteroatoms besides nitrogen and/or may be fused to an additional ring system; or Z is of the formula 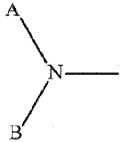
wherein A is a monocyclic or bicyclic fused ring system containing at least one N; and
B is H or an organic moiety of 1 to 20 atoms,
Z' is either Z or of the formula,

   -N(R)-(CR₂)n-X
wherein each R is independently H or straight, branched or cyclic alkyl (1-6C),
n is 1 or 2; and
X is an aromatic ring, including heteroaromatic rings, or is a mercaptan; and "linker" represents a bond, alkylene (1-6C) or may comprise aryl, fused aryl, and/or oxygen atoms contained in an alkylene chain, and/or may contain keto groups and/or nitrogen or sulfur atoms.

Suitable CXCR4 antagonists for the instantly disclosed methods and compositions also include the compounds of the formula

Z-R-A-R'-Y

or a pharmaceutically acceptable salt or metal complex thereof, in which Z and Y are identical cyclic polyamine moieties having from 9 to 20 ring members and from 3 to 6 amine nitrogens in the ring spaced by 2 or more carbon atoms from each other, A is an aromatic or heteroaromatic moiety other than quinoline, and R and R' are each methylene linked to an amine nitrogen atom in Z and in Y, the amine nitrogens being otherwise unsubstituted.

In the above formula, the cyclic polyamine moieties may be substituted or unsubstituted, and suitable substituents are alkyl and/or aryl groups, *e.g.,* of up to 10 carbon atoms, and any other atoms or groups which do not substantially adversely affect the activity or toxicity of the compounds. In certain embodiments, the cyclic polyamine moieties have 10 to 15 ring members and 3 or 4 amine nitrogen atoms in the ring.

The aromatic or heteroaromatic moiety A tethers Y and Z through the linking groups R and R'. Moiety A may be phenyl or fused aromatic such as naphthyl, heterocyclic such as pyridyl or thiophenyl, fused heterocyclic or joined aromatic and/or joined heteroaromatic, for example biphenyl or bipyridyl respectively. The moieties A may also be substituted at single or multiple non-linking positions with electron-donating groups, *e*.*g*., alkyl, thio, thioalkyl, hydroxyl, alkoxyl, amino and derivatives thereof, or electron-withdrawing groups or atoms, eg nitro, halogen, carboxy, carboxamido, sulfonic acid and derivatives thereof.

Suitable CXCR4 antagonists for the instantly disclosed methods and compositions also include the compounds of the formula

Z-R-A'-R'-Y (la)

or a pharmaceutically acceptable salt or metal complex thereof, in which Z, Y, R and R' are as defined above, with R and R' linked to nitrogen atoms in Z and Y, and A' is an aromatic or heteroaromatic moiety which is un substituted or substituted, other than quinoline, provided that A' is not unsubstituted phenylene when Z and Y are 14-membered tetraaza rings, and their acid addition salts and metal complexes.

Suitable CXCR4 antagonists for the instantly disclosed methods and compositions also include the compounds of formula

Z-R-A-R'-Y

in which Z and Y are identical cyclic polyamine moieties having from 10 to 15 ring members and from 3 to 4 amine nitrogens in the ring spaced by 2 or more carbon atoms from each other, said amine nitrogens being the only ring heteroatoms,

A is an unsubstituted aromatic or heteroaromatic moiety other
than quinoline;
R and R' are each methylene linked to nitrogen atoms in Z and Y, the amine nitrogen atoms being otherwise unsubstituted.

Suitable CXCR4 antagonists for the instantly disclosed methods and compositions also include the CXCR4 antagonist BKT140, including those CXCR4 antagonists described in U.S. Patent No. 7,423,007 and U.S. Patent Application Publication No. 2004/0171552; AYR 118; NIBR-1816; TG-0054, including those CXCR4 antagonists described in U.S. Patent No. 7,399,776 and U.S. Patent Publication Nos. 2006/0160860 and 2008/0058382; MSX-122; or POL-6326/ POL-2438/ POL-3026, including those CXCR4 antagonists described in WO 2008/104090. In certain embodiments, the antagonist may be an antibody, such as a monoclonal antibody, or immunoreactive fragment thereof.

Suitable CXCR4 antagonists for the instantly disclosed methods and compositions also include the CXCR4 antagonists, but are not limited to, linear peptides, cyclic peptides, natural amino acids, unnatural amino acids, and peptidomimetic compounds. Examples of such compounds include T22 (Murakami, T., et al., J. Exp. Med. (1997) 186:1389-1393); T134 (Arakaki, R,, e/a/., ,7 Virol. (1999) 73:1719-1723; T140 (Tamamura, H., et al., Biochem. Biophys. Res. Comm. (1998) 253:877-882) and its analogs TC14012 and TN14003 as shown in PCX Publication No. WO 2008/008854 (Tamamura, H., et al., Bioorg. Med. Chem. Lett. (2001) 11:1897-1902; Mori, X., etal., Mol. Cancer Ther. (2004) 3:29-37; Burger, M. , et al.. Blood (2005) 106:1824-1830); ALX40-4C (Doranz, B.J., et al., J. Exp. Med. (1997) 186:13951400; Donzella, G.A., Nat. Med. (1998) 4:72-77; Doranz, B.J., et al., AIDS Res. Hum. Retrovir. (2001) 17:475-486); RCP168 (Zeng, Z., et al., Mol. Cancer Ther. (2006) 5:3113-3121); CTCE-0021 (Pelus, L.M., etal., Exp. Hematol. (2005) 33:295-307); CTCE-0214 and CTCE-9908 (Zhong, R., et al., Exp. Hematol. (2004) 32:470-475; Kim, S.Y., et al., AACR Meeting Abstracts (2005) Abstract 256; PCX Publication Nos. WO 01/76615 and WO 01/85196; U.S. Patent Publication No. 2007/0160574 and related applications); KRH-1120 (Yamamoto, N. , et al., J. AIDS Res. (2000) 2:453-460); KRH-1636 (Ichiyama, K., et al., Proc. Natl. Acad. Sci. USA (2003) 100:4185-4190); KRH-2731/CS-3955 (Murakami T,, et al.. Abstracts of the 11th Conference on Retroviruses and Opportunistic Infections (2004) Abstract 541; PCX Publication Nos. WO 06/095542 and WO 02/094261); and CXCR4 antagonists described in PCX Publication Nos. WO 99/47158; WO 99/50461; WO 00/09152; WO 01/94420; and WO 03/090512.

Notable CXCR4 antagonist compounds disclosed herein are the following:
3,3 '-bis-1,5,9,13 -tetraazacyclohexadecane;
3,3 '-bis-1,5,8,11,14-pentaazacyclohexadecane;
5,5'-bis-1,4,8,11-tetraazacyclotetradecane;
2,5'-bis-1,4,8,11-tetraazacyclotetradecane;
2,6'-bis-1,4,8,11-tetraazacyclotetradecane;
methylene (or polymethylene) di 1-N-1,4,8,11-tetraazacyclotetradecane;
11,11'-(1,2-ethanediyl)bis-1,4,8,11-tetraazacyclotetradecane;
11,11'-(1,2-propanediyl)bis-1,4,8,11-tetraazacyclotetradecane;
11,11'-(1,2-butanediyl)bis-1,4,8,11-tetraazacyclotetradecane;
11,11'-(1,2-pentanediyl)bis-1,4,8,11-tetraazacyclotetradecane;
11,11'-(1,2-hexanediyl)bis-1,4,8,11-tetraazacyclotetradecane;
1,1'-[1,3-phenylene-bis(methylene)]-bis-1,4,8,11-tetraazacyclotetradecane;
1,1'-[1,4-phenylene-bis(methylene)]-bis-1,4,8,11-tetraazacyclotetradecane ;
1,1'-[3,3'-biphenylene-bis-(methylene)]-bis-1,4,8,11-tetraazacyclotetradecane;
11,11'-[1,4-phenylene-bis-(methylene)]-bis-1,4,7,11-tetraazacyclotetradecane;
1.11'-[1,4-phenylene-bis(methylene)]-1,4,8,11-tetraazacyclotetradecane;
1,1'-[2,6-pyridine-bis-(methylene)]-bis-1,4,8,11-tetraazacyclotetradecane;
1,1-[3,5-pyridine-bis-(methylene)]-bis-1,4,8,11-tetraazacyclotetradecane;
1,1'-[2,5-thiophene-bis-(methylene)]-bis-1,4,8,11-tetraazacyclotetradecane;
1,1'-[4,4'-(2,2'-bipyridine)-bis-(methylene)]-bis-1,4,8,11-tetraazacyclotetradecane;
1,1'-[2,9-(1,10-phenantbroline)-bis-(methylene)]-bis-1,4,8,11-tetraazacyclotetradecane;
1,1'-[1,3-phenylene-bis-(methylene)]-bis-1,4,7,10-tetraazacyclotetradecane;
1,1'-[1,4-phenylene-bis-(methylene)]-bis-1,4,7,10-tetraazacyclotetradecane;
1,1'-[5-nitro-1,3-phenylenebis(methylene)]bis-1 4,8,11-tetraazacyclotetradecane ;
1,1'-[2,4,5,6-tetrachloro-1,3-phenyleneis(methylene)]bis-1,4,8,11-tetraazacyclotetradecane;
1,1'-[2,3,5,6-tetrafluoro-1,4-phenylenebis(methylene)]bis-1,4,8,11-tetraazacyclotetradecane;
1,1'-[1,4-naphthylene-bis-(methylene)]bis-1,4,8,11-tetraazacyclotetradecane;
1,1'-[1,3-phenylenebis-(methylene)]bis-1,5,9-triazacyclododecane;
1,1'-[1,4-phenylene-bis-(methylene)]-1,5,9-triazacyclododecane;
1,1'-[2,5-dimethyl-1,4-phenylenebis-(methylene)]-bis-1,4,8,11-tetraazacyclotetradecane;
1,1'-[2,5-dichloro-1,4-phenylenebis-(methylene)]-bis-1,4,8,11-tetraazacyclotetradecane;
1,1'-[2-bromo-1,4-phenylenebis-(methylene)]-bis-1,4,8,11-tetraazacyclotetradecane;
1,1'-[6-phenyl-2,4-pyridinebis-(methylene)]-bis-1,4,8,11-tetraazacyclotetradecane;
7,7'-[1,4-phenylene-bis(methylene)]bis-3,7,11,17-tetraazabicyclo[13.3.1]heptadeca-1(17),13,15 triene;
7,7'-[1,4-phenylene-bis(methylene)]bis[15-chloro-3,7,11,17-tetraazabicyclo[13.3.1]heptadeca1(17),13,15-triene];
7,7'-[1,4-phenylene-bis(methylene)]bis[15-methoxy-3,7,11,17-tetraazabicyclo[13.3.1]heptadeca1(17),13,15-triene];
7,7'-[1,4-phenylene-bis(methylene)]bis-3,7,11,17-tetraazabicyclo [13.3.1]-heptadeca-13,16-triene 15-one;
7,7'-[1,4-phenylene-bis(methylene)]bis-4,7,10,17-tetraazabicyclo[13.3.1]-heptadeca-1(17),13,15 triene;
8,8'-[1,4-phenylene-bis(methylene)]bis-4,8,12,19-tetraazabicyclo[15.3.1]nonadeca-1 (19), 15,17triene;
6,6'-[1,4-phenylene-bis(methylene)]bis-3,6,9,15-tetraazabicyclo[11.3.1]pentadeca-1(15),11,13triene;
6,6'-[1,3-phenylene-bis(methylene)]bis-3,6,9,15-tetraazabicyclo[11.3.1]pentadeca-1(15),11,13triene;
17,17'-[1,4-phenylene-bis(methylene)]bis-3,6,14,17,23,24-hexaazatricyclo[17.3.1.18,12]tetracosal(23),8,10,12(24),19,21-hexaene;
N-[1,4,8,11-tetraazacyclotetradecanyl-1,4-phenylenebis(methylene)]-2-(amino-methyl)pyridine ;
N-[1,4,8,11-tetraazacyclotetradecanyl-1,4-phenylenebis(methylene)]-N-methyl-2(aminomethyl)pyridine;
N-[1,4,8,11-tetraazacyclotetradecanyl-1,4-phenylenebis(methylene)]-4-(amino-methyl)pyridine;
N-[1,4,8,11 -tetraazacyclotetradecanyl-1,4-phenylenebis(methylene)]-3-(amino-methyl)pyridine ;
N-[1,4,8,11-tetraazacyclotetradecanyl-1,4-phenylenebis(methylene)]-(2-amino-methyl-5methyl)pyrazine;
N-[1,4,8,11-tetraazacyclotetradecanyl-1,4-phenylenebis(methylene)]-2-(amino-ethyl)pyridine;
N-[1,4,8,11-tetraazacyclotetradecanyl-1,4-phenylenebis(methylene)]-2-(amino-methyl)thiophene;
N-[1,4,8,11-tetraazacyclotetradecanyl-1,4-phenylenebis(methylene)]-2-(amino-ethyl)mercaptan;
N-[1,4,8,11-tetraazacyclotetradecanyl-1,4-phenylenebis(methylene)]-2-amino-benzylamine;
N-[1,4,8,11-tetraazacyclotetradecanyl-1,4-phenylenebis(methylene)]-4-amino -benzylamine;
N-[1,4,8,11-tetraazacyclotetradecanyl-1,4-phenylenebis(methylene)]-4-(amino-ethyl)imidazole;
N-[1,4,8,11-tetraazacyclotetradecanyl-1,4-phenylenebis(methylene)]-benzylamine;
N-[1,4,8,11-tetraazacyclotetradecanyl-1,4-phenylenebis(methylene)]-purine;
N-[1,4,8,11 -tetraazacyclotetradecanyl-1,4-phenylenebis(methylene)]-4-phenylpiperazine;
1-[2,6-dimethoxypyrid-4-yl(methylene)]-1,4,8,11-tetraazacyclotetradecane;
1-[2-chloropyrid-4-yl(methylene)] -1,4,8,11 -tetraazacyclotetradecane;
1-[2,6-dimethylpyrid-4-yl(methylene)]-1,4,8,11-tetraazacyclotetradecane;
1-[2-methylpyrid-4-yl(methylene)]-1,4,8,11-tetraazacyclotetradecane;
1-[2,6-dichloropyrid-4-yl(methylene)]-1,4,8,11-tetraazacyclotetradecane;
1-[2-chloropyrid-5-yl(methylene)]-1,4,8,11-tetraazacyclotetradecane;
7-[4-methylphenyl (methylene)]-4,7,10,17-tetraazabicyclo[13.3.1]heptadeca-1(17),13,15-triene;
N-[4-(1,4,7-triazacyclotetra-decanyl)-1,4-phenylenebis(methylene)]-2-(aminomethyl)pyridine;
N-[1-(1,4,7-triazacyclotetra-decanyl)-1,4-phenylenebis(methylene)]-2-(aminomethyl)pyridine;
N-[7-(4,7,10,17-tetraazabicyclo[13.3.1]heptadeca-1(17),13,15-trienyl)-1,4phenylenebis(methylene)]-2-(aminomethyl)pyridine;
N-[7-(4,7,10-triazabicyclo[13.3.1]heptadeca-1(17),13,15-trienyl)-1,4-phenylenebis(methylene)]-2(aminomethyl)pyridine;
N-[4-[4,7,10-triazabicyclo[13.3.1]heptadeca-1(17),13,15-trienyl]-1,4-phenylenebis(methylene)]-2(aminomethyl)pyridine;
N-[4-[4,7,10,17-tetraazabicyclo[13.3.1]heptadeca-1(17),13,15-trienyl]-1,4phenylenebis(methylene)]-2-(aminomethyl)pyridine;
N-[3-(3,6,17-triazabicyclo[13.3.1]heptadeca-1(17),13,15-trienyl)-1,4-phenylenebis(methylene)]-2(aminomethyl)pyridine;
N-[3-(3,6,17-triazabicyclo[13.3.1]heptadeca-1(17),13,15-trienyl)-1,3-phenylenebis(methylene)]-2(aminomethyl)pyridine;
N-[4-(4,7,17-triazabicyclo[13.3.1]heptadeca-1(17),13,15-trienyl)-1,4-phenylenebis(methylene)]-2(aminomethyl)pyridine;
N-[7-(4,7,17-triazabicyclo[13.3.1]heptadeca-1(17),13,15-trienyl)-1,4-phenylenebis(methylene)]-2(aminomethyl)pyridine;
N-[6-(3,6,9-triazabicyclo[11.3.1]pentadeca-1(15),11,13-trienyl)-1,3-phenylenebis(methylene)]-2(aminomethyl)pyridine;
N-[4-(1,7-diazacyclotetradecanyl)-1,4-phenylenebis(methylene)]-2-(aminomethyl)pyridine;
N-[7-(4,10-diazabieyclo[13.3.1]heptadeca-1(17),13,15-trienyl)-1,4-phenylenebis(methylene)]-2(aminomethyl)pyridine;
N-[7-(4,10,17-triazabicyclo[13.3.1]heptadeca-1(17),13,15-trienyl)-1,4-phenylenebis(methylene)]2-(aminomethyl)pyridine;
N-[4-(11-fluoro-1,4,7-triazacyclotetradecanyl)-1,4-phenyl enebis(methylene)]-2(aminomethyl)pyridine;
N-[4-(11,11-difluoro-1,4,7-triazacyclotetradecanyl)-1,4-phenylenebis(methylene)]-2(aminomethyl)pyridine;
N-[4-(1,4,7-triazacyclotetradecan-2-onyl)-1,4-phenylenebis(methylene)]-2-(aminomethyl)pyridine;
N-[12-(5-oxa-1,9-diazacyclotetradecanyl)-1,4-phenylenebis(methylene)]-2-(aminomethyl)pyridine;
N-[4-(11-oxa-1,4,7-triazacyclotetradecanyl)-1,4-phenylenebis(methylene)]-2(aminomethyl)pyridine;
N-[4-(11-thia-1,4,7-triazacyclotetradecanyl)-1,4-phenylenebis(methylene)]-2(aminomethyl)pyridine;
N-[4-(11-sulfoxo-1,4,7-triazacyclotetradecanyl)-1,4-phenylenebis(methylene)]-2(aminomethyl)pyridine;
N-[4-(11-sulfono-1,4,7-triazacyclotetradecanyl)-1,4-phenylenebis(methylene)] -2(aminomethyl)pyridine; and
N-[4-(3-carboxo-1,4,7-triazacyclotetradecanyl)-1,4-phenylenebis(methylene)]-2(aminomethyl)pyridine,
or a pharmaceutically acceptable salt or metal complex thereof.

### Methods for Making the CXCR4 Antagonists:

As noted above, one of the applicants has previously disclosed methods for preparing certain of the presently disclosed CXCR4 antagonists, including plerixafor, in U.S. Patent Nos. 5,612,478; 5,756,728; 5,801,281; and 5,606,053; 6,489,472 and WO 02/26721. Those of skill in the art given the benefit of the instant application and applicant's previously disclosed application will be readily able to make the CXCR4 antagonists disclosed herein, including those of formula I.

### Active Pharmaceutical Ingredient fAPI) Derivatives:

All pharmaceutically acceptable salts, prodrugs, tautomers, hydrates and solvates of the compounds presently disclosed are also disclosed herein. Presently disclosed compounds that are basic in nature are generally capable of forming a wide variety of different salts with various inorganic and/or organic acids. Although such salts are generally pharmaceutically acceptable for administration to animals and humans, it is often desirable in practice to initially
isolate a compound from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds can be readily prepared using conventional techniques, e.g., by treating the base compound with a substantially equivalent amount of the chosen mineral or organic acid in an aqueous solvent medium or in a suitable organic solvent such as, for example, methanol or ethanol. Upon careful evaporation of the solvent, the desired solid salt is obtained.

Acids which can be used to prepare the pharmaceutically acceptable acid addition salts of the base compounds are those which can form non-toxic acid addition salts, *i.e*., salts containing pharmacologically acceptable anions, such as chloride, bromide, iodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate *[i.e.,* 1,1'-methylene-bis-(2-hydroxy-3-naphthoate)] salts. Presently disclosed compounds that are acidic in nature, e.g., contain a COOH or tetrazole moiety, are generally capable of forming a wide variety of different salts with various inorganic and/or organic bases. Although such salts are generally pharmaceutically acceptable for administration to animals and humans, it is often desirable in practice to initially isolate a compound from the reaction mixture as a pharmaceutically unacceptable salt and then simply convert the latter back to the free acid compound by treatment with an acidic reagent, and subsequently convert the free acid to a pharmaceutically acceptable base addition salt. These base addition salts can be readily prepared using conventional techniques, e.g., by treating the corresponding acidic compounds with an aqueous solution containing the desired pharmacologically acceptable cations, and then evaporating the resulting solution to dryness, in certain embodiments under reduced pressure. Alternatively, they also can be prepared by mixing lower alkanolic solutions of the acidic compounds and the desired alkali metal alkoxide together, and then evaporating the resulting solution to dryness in the same manner as before. In either case, stoichiometric quantities of reagents are employed in order to ensure completeness of reaction and maximum product yields of the desired solid salt.

Bases which can be used to prepare the pharmaceutically acceptable base addition salts of the base compounds are those which can form non-toxic base addition salts, *i.e*., salts containing pharmacologically acceptable cations, such as, alkali metal cations *{e.g*., potassium and sodium), alkaline earth metal cations (*e.g*., calcium and magnesium), ammonium or other water-soluble amine addition salts such as N-methylglucamine-(meglumine), lower alkanolammonium and other such bases of organic amines.

Non-toxic labile metal complexes of the presently disclosed compounds are also active compounds, disclosed herein. Non-toxic in the present context has to be considered with reference to the prognosis for the infected patient without treatment. Copper and zinc complexes are notable metal complexes although other metals such as nickel may be considered. Less labile metal atoms such as cobalt and rhodium may exhibit lower selectivity as compared to copper and zinc.

Isotopically-labeled compounds are also disclosed herein. As used herein, an "isotopically-labeled compound" refers to a presently disclosed compound including pharmaceutical salts and prodrugs thereof, each as described herein, in which one or more atoms are replaced by an atom having an atomic mass or mass number different from the atomic mass or mass number usually found in nature. Examples of isotopes that can be incorporated into compounds presently disclosed include isotopes of hydrogen, carbon, nitrogen, oxygen, phosphorous, fluorine and chlorine, such as ²H, ³H, ¹³C, ¹⁴C, ¹⁵N, ¹⁸0, ¹⁷0, ³¹P, ³²P, ³⁵S, ¹⁸F, and ³⁶Cl, respectively. By isotopically-labeling the presently disclosed compounds, the compounds may be useful in drug and/or substrate tissue distribution assays. Tritiated (³H) and carbon-14 (¹⁴C) labeled compounds are particularly preferred for their ease of preparation and detectability. Further, substitution with heavier isotopes such as deuterium (²H) can afford certain therapeutic advantages resulting from greater metabolic stability, for example increased in vivo half-life or reduced dosage requirements and, hence, may be preferred in some circumstances. Isotopically labeled compounds presently disclosed, including pharmaceutical salts and prodrugs thereof, can be prepared by any means known in the art.

Stereoisomers *{e.g*., cis and trans isomers) and all optical isomers of a presently disclosed compound *(e.g*., R and S enantiomers), as well as racemic, diastereomeric and other mixtures of such isomers are disclosed herein.

The compounds, salts, prodrugs, hydrates, and solvates presently disclosed can exist in several tautomeric forms, including the enol and imine form, and the keto and enamine form and geometric isomers and mixtures thereof. Tautomers exist as mixtures of a tautomeric set in solution. In solid form, usually one tautomer predominates. Even though one tautomer may be described, all tautomers are disclosed herein. Atropisomers are also disclosed herein. Atropisomers refer to compounds that can be separated into rotationally restricted isomers.

### Dosing:

The CXCR4 antagonists disclosed herein may be administered for the treatment or prevention of WHIM syndrome and certain other disorders or conditions as a single bolus dose, a dose over time, or in multiple dosages. In certain embodiments, each unit dose of a CXCR4 antagonist disclosed herein or a pharmaceutical composition comprising such CXCR4 antagonist is administered as a variable amount depending on the weight of the human patient, the route of administration and other factors. A representative unit dose, particular for plerixafor, is a single-use vial for subcutaneous administration containing 1.2 mL of a 20 mg/mL solution. Here, the 20 mg/mL solution is a strength of the dose in which there is 20 mg of the CXCR4 antagonist (API) in 1 mL of solution.

Suitable dosage ranges for the CXCR4 antagonists for the treatment or prevention of WHIM syndrome and a disorder or condition disclosed herein vary according to these considerations, but in general, the compounds are administered in the range of about 0.01 pg/kg-10 mg/kg of body weight. In certain embodiments, especially where the CXCR4 antagonist is dosed parenterally, the dose range of the CXCR4 antagonist is 0.02 mg/kg to 0.24 mg/kg. For a typical 70-kg human subject, thus, the dosage range is from about 2.8 mg to about 16.8 mg. In certain embodiments, the dose range of the CXGR4 antagonist is 0.04 mg/kg to 0.24 mg/kg. In certain embodiments, the dose range of the CXCR4 antagonist is 0.02 mg/kg to 0.72 mg/kg. In certain embodiments, the CXCR4 antagonist is dosed at 0.02 mg/kg, 0.04 mg/kg, 0.08 mg/kg, 0.24 mg/kg, 0.4 8 mg/kg, 0.72 mg/kg. In certain embodiments, the CXCR4 antagonist is dosed at, 0.01 mg/kg, 0.02 mg/kg, 0.03 mg/kg, 0.04 mg/kg, 0.05 mg/kg, 0.06 mg/kg, 0.07 mg/kg, 0.08 mg/kg, 0.09 mg/kg, 0.10 mg/kg, 0.11 mg/kg, 0.12 mg/kg, 0.13 mg/kg, 0.14 mg/kg, 0.15 mg/kg, 0.16 mg/kg, 0.17 mg/kg, 0.18 mg/kg, 0.19 mg/kg, 0.20 mg/kg, 0.21 mg/kg, 0.22 mg/kg, 0.23 mg/kg, 0.24 mg/kg, 0.48 mg/kg, 0.72 mg/kg.

In certain embodiments, especially where the CXCR4 antagonist is dosed orally for the treatment or prevention of WHIM syndrome and a disorder or condition disclosed herein is about 0.1 mg to about 2000 mg. In certain embodiments, the proposed dose is from about 0.1 mg to about 200 mg of the active ingredient per unit dose.

Irrespective of the amount of the dose of the CXCR4 antagonist for the treatment or prevention of WHIM syndrome and a disorder or condition disclosed herein, the compound or composition can occur every other day (QOD), once daily (QD), twice daily (BID), three times daily (TID) or four times daily (QID), where the dose can be the same or different during each of these intervals. For example, in a BID dosing regimen, a composition comprising the CXCR4 antagonist can be dosed at 0.24 mg/kg during the first administration and 0.48 mg/kg during the second administration that day.

In certain embodiments, the CXCR4 antagonist disclosed herein is used for the chronic treatment of WHIM syndrome or a disorder or condition disclosed herein. As explained herein, a patient with myelokathexis, for instance, generally has poor bone marrow health. A chronic treatment of plerixafor, for instance, may be needed to obtain and retain the improved marrow health achieved with the drug. In accordance with chronic treatment, a patient is continually administered a CXCR4 antagonist, such as plerixafor, in order to maintain the benefits achieved with the drug (e.g., improved bone marrow health).

In alternative embodiments, acute treatment may be possible to treat a patient with WHIM syndrome or a disorder or condition disclosed herein. In accordance with these embodiments, a patient with, e.g., myelokathexis, is initially treated with, for instance, plerixafor to improve the marrow health by clearing the dead and dying neutrophils and other cells from the marrow. Once treatment is ceased after the patient achieves normal hematopoiesis, such normal hematopoiesis is maintained without further plerixafor treatment.

In addition, the CXCR4 antagonist can be dosed in a range of 1 to 10 days in accordance of one or more of the regimens discussed immediately above. In certain embodiments, the CXCR4 antagonist is dosed for 2 to 4 days. In these embodiments, the CXCR4 antagonist is dosed QD for 2 to 4 days.

It is thus apparent that the required dosage will of course vary depending on the mode of administration, the particular condition to be treated and the effect desired. Those of skill in the art given the benefit of the instant application will be able to devise a dosage regimen that will be suitable to treat a human patient with WHIM syndrome and a disorder or condition disclosed herein.

### Route of Administration:

The route of administration chosen will be tailored to the individual subject or patient, the nature of the condition to be treated in the subject, and generally, the judgment of the attending practitioner.

In certain embodiments, the compounds and pharmaceutical compositions disclosed herein are administered by a parenteral route, *e*.*g*., via intramuscular, intraperitoneal, intravenous, intracisternal, intraarticular, or subcutaneous injection or infusion. One notable CXCR4 antagonist disclosed herein, plerixafor, is typically administered by a subcutaneous route, although it is sometimes administered intravenously in a clinical setting. In accordance with chronic treatment, plerixafor can be administered by a continuous infusion.

Besides injection, other routes of administration may also be used. In certain embodiments, the compounds and pharmaceutical compositions disclosed herein are administered by an oral route. In certain embodiments, the compounds and pharmaceutical compositions disclosed herein are administered by a topical or transdermal route.

### Pharmaceutical Compositions:

Those of skill in the art given the benefit of the instant application will appreciate that an appropriate pharmaceutical composition comprising a CXCR4 antagonist disclosed herein can be suitably formulated based on the route of administration chosen by the attending practitioner.

Such pharmaceutical compositions will comprise at least one presently disclosed compound and at least one pharmaceutically acceptable diluent or carrier. Pharmaceutical compositions of the compounds presently disclosed may be prepared by conventional means known in the art including, for example, mixing at least one presently disclosed compound with a pharmaceutically acceptable diluent or carrier. The CXCR4 antagonists may be formulated for administration to the subject or patient using commonly understood formulation techniques well known in the art. Formulations which are suitable for particular modes of administration and for compounds useful in the disclosure may be found in Remington's The Science and Practice of Pharmacy. 21st edition, Lippincott Williams & Wilkins, Hagerstown, MD.

Presently disclosed pharmaceutical compositions are intended to be for human use in a patient. Thus, a presently disclosed compound can be formulated as a pharmaceutical composition for any route of administration disclosed herein, including oral, buccal, parenteral (e.g., intravenous, intramuscular or subcutaneous), topical, rectal or intranasal administration or in a form suitable for administration by inhalation or insufflation. The compounds presently disclosed may also be formulated for sustained delivery according to methods well known to those of ordinary skill in the art. Examples of such formulations can be found in U.S Patent Nos. 3,119,742, 3,492,397, 3,538,214, 4,060,598, and 4,173,626.

For oral administration, the pharmaceutical composition may take the form of, for example, a tablet or capsule prepared by conventional means with a pharmaceutically acceptable excipient(s) such as a binding agent *(e*.*g*., pregelatinized maize starch, polyvinylpyrrolidone or hydroxypropyl methylcellulose); filler *(e.g*., lactose, microcrystalline cellulose or calcium phosphate); lubricant *(e*.*g*., magnesium stearate, talc or silica); disintegrant *(e*.*g*., potato starch or sodium starch glycolate); and/or wetting agent *(e*.*g*., sodium lauryl sulphate). The tablets may be coated by methods well known in the art. Liquid preparations for oral administration may take the form of a, for example, solution, syrup or suspension, or they may be presented as a dry product for constitution with water or other suitable vehicle before use. Such liquid preparations may be prepared by conventional means with a pharmaceutically acceptable additive(s) such as a suspending agent *(*e.g., sorbitol syrup, methyl cellulose or hydrogenated edible fats); emulsifying agent *(e.g.,* lecithin or acacia); non-aqueous vehicle *(e.g.,* almond oil, oily esters or ethyl alcohol); and/or preservative *(e.g.,* methyl or propyl p-hydroxybenzoates or sorbic acid).

For buccal administration, the composition may take the form of tablets or lozenges formulated in a conventional manner.

Presently disclosed compounds may be formulated for parenteral administration by injection, including using conventional catheterization techniques or infusion. Formulations for injection may be presented in unit dosage form, *e.g.,* in vials, ampules or in multi-dose containers, with an added preservative. The compositions may take such forms as suspensions, solutions or emulsions in oily or aqueous vehicles, and may contain a formulating agent such as a suspending, stabilizing and/or dispersing agent recognized by those of skill in the art. The compositions may contain liposomes or other suitable carriers. For injection intravenously, the solution is made isotonic using standard preparations such as Hank's solution. Alternatively, the active ingredient may be in powder form for reconstitution with a suitable vehicle, e.g., sterile pyrogen-free water, before use. One notable CXCR4 antagonist disclosed herein, plerixafor, is typically presented in a single-use vial containing 1.2 mL of a 20 mg/mL solution in which the vial contains 24 mg of plerixafor and 5.9 mg of sodium chloride in water for injection adjusted to a pH of 6.0 to 7.5 with hydrochloric acid and with sodium hydroxide, if required.

For topical administration, a presently disclosed CXCR4 antagonist may be formulated as an ointment or cream.

Presently disclosed CXCR4 antagonists may also be formulated in rectal compositions such as suppositories or retention enemas, *e.g.,* containing conventional suppository bases such as cocoa butter or other glycerides.

For intranasal administration or administration by inhalation, presently disclosed CXCR4 antagonists may be conveniently delivered in the form of a solution or suspension from a pump spray container that is squeezed or pumped by the patient or as an aerosol spray presentation from a pressurized container or a nebulizer, with the use of a suitable propellant, *e.g.,* dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the presently disclosed compound. Capsules and cartridges (made, for example, from gelatin) for use in an inhaler or insufflator may be formulated containing a powder mix of a presently disclosed compound and a suitable powder base such as lactose or starch.

Aerosol combination formulations for the treatment or prevention of the conditions referred to above in the average adult human are arranged so that each metered dose or "puff' of aerosol contains from about 0.01 mg to about 1000 mg of a presently disclosed compound. Administration may be several times daily, for example 2, 3, 4 or 8 times, giving for example, 1, 2 or 3 doses each time.

### Methods:

As discussed above, the compounds and compositions disclosed herein are useful for treating or preventing WHIM syndrome or a disease or condition associated with WHIM syndrome in a human. Such methods are performed by administering to a human, typically a patient, a CX CR4 antagonist in an amount effective to treat or prevent WHIM syndrome.

In certain embodiments, the CXCR4 antagonist used to treat or prevent WHIM syndrome or a disease or condition associated with W HIM syndrome is a compound of formula I,

Z-linker-Z' (I)

or a pharmaceutically acceptable salt or metal complex thereof, wherein
Z is a cyclic polyamine containing 9-32 ring members of which 2-8 are nitrogen atoms, said nitrogen atoms being separated from each other by at least 2 carbon atoms, and wherein said heterocycle may optionally contain additional heteroatoms besides nitrogen and/or may be fused to an additional ring system;
Z' is either Z or of the formula

   -N(R)-(CR₂)n-X

   wherein each R is independently H or straight, branched or cyclic alkyl (1-6C), n is 1 or 2, and X is an aromatic ring, including heteroaromatic rings, or is a mercaptan, or Z' is of the formula

   -Ar(Y)j

   wherein Ar is an aromatic or heteroaromatic moiety, and each Y is independently a non-interfering substituent and j is 0-3; and
   "linker" represents a bond, alkylene (1-6C) or may comprise aryl, fused aryl, oxygen atoms contained in an alkylene chain, or may contain keto groups or nitrogen or sulfur atoms.

CXCR4 antagonists suitable for use in the presently disclosed methods are cyclic polyamines owned by one of the present applicants, Genzyme Corporation. A notable cyclic polyamine is plerixafor (formerly known as AMD3100), which is disclosed in U.S. Patent No. RE42,152. Mozobil® (plerixafor), was approved by the United States FDA in 2008 for use in combination with granulocyte-colony stimulating factor (G-CSF) to mobilize hematopoietic stem cells to the peripheral blood for collection and subsequent autologous transplantation in patients with non-Hodgkin's lymphoma and multiple myeloma. This FDA approved use, which is generally described as an autologous hematopoietic stem cell transplant, is disclosed in U.S. Patent Nos. 6,987,102 and 7,897,590. The inventors here have discovered that these CXCR4 antagonists, including plerixafor, are useful for WHIM syndrome or a disease or condition associated with WHIM syndrome. This discovery is not based on mere speculation or prophecy. Rather, this discovery is predicated on recently obtained clinical data from a study conceived and designed by the inventors, as discussed in Examples section herein.

Those of skill in the art given the benefit of the instant application will appreciate the signs to determine the effects of the CXCR4 antagonist on a patient having WHIM syndrome or myelokathexis. For example, a patient with WHIM syndrome may have numerous warts on the hands and/or feet to such a degree that walking or grasping, as the case may be, is difficult, if not impossible. A skilled physician may observe the effect of administering a CXCR4 antagonist to such a patient by noticing that the warts are less prevalent after a suitable dosing schedule, that there is a reduction in the number and severity of infections over time, a reduction in disease-associated hospitalizations or physician visits, a reduction in the need for anti-infective therapy intervention, improvement in immunoglobulin levels, improved patient weight and/or an improvement in overall patient health and feeling of well-being.

The CXCR4 antagonists disclosed herein are useful for treating or preventing certain other disorders or conditions such as hypogammaglobulinemia, myelokathexis, neutropenia, leukopenia, immunodeficiency diseases associated with B, T, or NK lymphopenia, abnormalities attributed to improper trafficking of leukocytes (cell proliferation and production), severe combined immunodeficiency syndrome (SCID's), diphtheria, bacterial infections, viral infections, or reduced immune function, and immunodeficiencies affecting trafficking of leukocytes. In a patient afflicted with WHIM syndrome, the white blood cell count (WBC) is usually <1.0 x 10⁹/L. with severe neutropenia and lymphocytopenia. Marrow examination shows abundant neutrophils with hyper-segmented nuclei and remnants of neutrophils in marrow macrophages.

Notably, the inventors have determined that with the use of a CXCR4 antagonist disclosed herein, it is possible to decrease bacterial infections independent of lymphocyte levels. Such a reaction has not been observed with any other drug. While not being bound by theory, it is believed that this phenomenon is a result of disrupting the CXCR4/SDF-1 interaction with a CXCR4 antagonist disclosed herein, such as plerixafor. Other uses of these CXCR4 antagonists include restoring normal cell trafficking of leukocytes from spaces such as the bone marrow into the peripheral blood circulation and then to areas where such cells are needed, for example lymph nodes and peripheral organs. These CXCR4 antagonists can also be used to correct neutropenia, lymphopenia, and leukopenias of other cell types (B, T, NK). A CXCR4 antagonist disclosed herein, such as plerixafor, that is administered to an immunodeficient patient will advantageously increase the number of B, T, and/or NK cells. The CXCR4 antagonists are also useful for reducing viral infections by increasing circulating T cells and NK cells and allowing trafficking of these cells to sites of infection. The CXCR4 antagonists are also useful for reducing bacterial infections by increasing circulating neutrophils/monocyte/macrophages and allowing trafficking to sites of infection. Reduction of viral and bacterial infections by restoring normal antibody production and gamma globulin levels thereby allowing protection against viral infection, improved bacterial opsonization/killing, protection against other pathogens/parasites with a CXCR4 antagonist disclosed herein is an envisioned use disclosed herein. With a CXCR4 antagonist disclosed herein, normal trafficking of B cells to immune sites such as draining lymph nodes and permitting critical interactions with new antigens occurs in the context of follicular dendritic cells, T cells and other supportive cells. These interactions are crucial for effective antibody generation and production in response to vaccines and infections. Improvement in immune function with the use of a CXCR4 antagonist disclosed herein may also improve anti-tumor surveillance and lead to a reduction in the potential development of cancer.

An unexpected benefit from the clinical study disclosed in the Examples section may be the establishment of improved marrow cell "health" and hematopoiesis. In this study, there was an initial large release of neutrophils and other cells from the bone marrow and other hematopoietic sites *{e.g*., lymph nodes, spleen) followed by a smaller incremental release of these cells with subsequent treatment of plerixafor over the next few days since the first dose of plerixafor followed by larger releases of cells. In normal humans, certain types of cells (neutrophils, lymphocytes and other cells) are removed from circulation by the spleen and liver and destroyed at the end of their normal life-span. In human patients afflicted with myelokathexis, these cell types (neutrophils, lymphocytes and other cells) are trapped in the bone marrow and thus do not get destroyed. This condition is especially troublesome in the case of neutrophils because they have a short life-span (with a normal half-life of about three days). Consequently, not being able to clear these dead or dying neutrophils from the marrow results in such cells remaining present in the marrow at high concentrations in patients with myelokathexis. Without being bound by theory, plerixafor is believed to release all of these cells *(e.g.*, neutrophils, leukocytes, both new and old) from the bone marrow in patients with myelokathexis. Upon the release of these cells into circulation, they are then removed and destroyed by normal mechanisms. With the removal of these old and dying cells, space in the bone marrow becomes available for normal growth and maturation of cells and the clearing of any dead or dying cell debris. The result would be a more normal appearing and functioning marrow and a general improvement in marrow cell "health" and hematopoiesis.

As observed in the clinical study disclosed herein, all patients had a mutation of the CXCR4 gene (R334 and S324fs365). These mutations are activating and prevent the normal release of neutrophils and other leukocytes from the marrow and other tissues and organs into the blood. As such, the probability for achieving effective treatment in a patient with WHIM syndrome or a disorder or condition disclosed herein is increased in a patient having these mutations of the CXCR4 gene (R334 and S324fs365). Many mutations of the CXCR4 gene are known, however, it is not understood whether all mutations of the CXCR4 gene will increase the propensity for a patient to achieve effective treatment in a patient with WHIM syndrome or a disorder or condition disclosed herein.

In accordance with the instantly disclosed methods, it would be advantageous to screen for patients to determine if they have the R334 and S324fs365 CX CR4 mutations. The details of performing such a screen are within the skill of one in the art. Patients having WHIM syndrome or a disease or condition disclosed herein that are determined to have the R334 and S324fs365 CXCR4 mutations are believed to have a higher probability for being treated for such a disorder or condition with a CXCR4 antagonist disclosed herein, such as plerixafor. Thus, such patients having these R334 and S324fs365 CXCR4 mutations are selected to be administered for CXCR4 antagonist treatment due to their increased chances of successful treatment.

### Use of CXCR4 Antagonists in Combination with Other Agents:

As disclosed herein, a CXCR4 antagonist disclosed herein is useful for treating or preventing WHIM syndrome (and a disorder or condition disclosed herein) in a patient in need thereof. Such therapies generally involve administering the CXCR4 antagonist as a monotherapy. Those of skill in the art given the benefit of the instant application will appreciate, however, that a human patient afflicted with WHIM syndrome and a disorder or condition disclosed herein may be treated with a course of therapy that comprises an additional pharmaceutically active ingredient *{i.e*., in addition to a CXCR4 antagonist). In these cases, a combination therapy may be suitable for treating or preventing such patients.

The term "pharmaceutical combination therapy" or "in combination" or just "combination therapy" as used herein generally refers to the administration of a CXCR4 antagonist in combination with one or more agents disclosed herein. In other words, the term "pharmaceutical combination therapy" means the CXCR4 antagonist, such as a compound of formula (I), may be administered concomitantly in a pharmaceutically acceptable form with one or more of the agents disclosed herein: (i) in the same dosage form, *e.g.,* the same tablet or pharmaceutical composition meaning a pharmaceutical composition comprising a CXCR4 antagonist, such as a compound of formula (I), one or more agents disclosed herein, and a pharmaceutically acceptable diluent or carrier; (ii) in a separate dosage form having the same mode of administration, *e.g*., *a* kit comprising a first pharmaceutical composition suitable for s.c. administration comprising a CXCR4 antagonist, such as a compound of formula (I) and a pharmaceutically acceptable diluent or carrier, and a second pharmaceutical composition suitable for s.c. administration comprising one or more agents disclosed herein and a pharmaceutically acceptable diluent or carrier; and (iii) in a separate dosage form having different modes of administration, e.g., a kit comprising a first pharmaceutical composition suitable for s.c. administration comprising a CXCR4 antagonist, such as a compound of formula (I) and a pharmaceutically acceptable diluent or carrier, and a second pharmaceutical composition suitable for oral administration comprising one or more agents disclosed herein and a pharmaceutically acceptable diluent or carrier. Further, those of skill in the art given the benefit of the present disclosure will appreciate that when more than one agents disclosed herein is being administered, the agent(s) need not share the same mode of administration, e.g., a kit comprising a first pharmaceutical composition suitable for s.c. administration comprising a CXCR4 antagonist, such as a compound of formula (I) and a pharmaceutically acceptable diluent or carrier, a second pharmaceutical composition suitable for oral administration comprising a first agent disclosed herein and a pharmaceutically acceptable diluent or carrier, and a third pharmaceutical composition suitable for parenteral administration comprising a second agent disclosed herein and a pharmaceutically acceptable diluent or carrier. Those of skill in the art will appreciate that the concomitant administration referred to above in the context of a "pharmaceutical combination therapy" or "combination therapy" means that the pharmaceutical composition comprising a CXCR4 antagonist and a pharmaceutical composition(s) comprising the agent can be administered on the same schedule, *i.e.,* at the same time and day, or on a different, schedule, *i,e.,* on different, although not necessarily distinct, schedules. In that regard, when the pharmaceutical composition comprising a CXCR4 antagonist and a pharmaceutical composition(s) comprising the agent is administered on a different schedule, such a different schedule may also be referred to herein as "background" or "background administration." For example, the pharmaceutical composition comprising CXCR4 antagonist may be administered at a certain dosage once a day, and the pharmaceutical composition(s) comprising the agent, *e.g.,* G-CSF, may be administered once a day for one or more days prior to the dose of the CXCR4 antagonist, such that the pharmaceutical composition comprising the CXCR4 antagonist may be administered at the same time as the pharmaceutical composition(s) comprising the agent during one of the daily administrations. Of course, other suitable variations to "pharmaceutical combination therapy" will be readily apparent to those of skill in the art given the benefit of the present disclosure and are part of the meaning of this term.

In certain embodiments, a presently disclosed CXCR4 antagonist is administered in combination with other drugs or agents in accordance with the methods disclosed herein. In certain embodiments, a presently disclosed CXCR4 antagonist is administered in combination with an anti-infective or antiinflammatory agent. For example, the compounds of formula I may be administered in combination with gammaglobulin, immunoglobulin, cytokines, *e.g.,* G-CSF, GM-CSF, IL-3, stem cell factor, flt-3 ligand, macrophage inflammatory protein.

In certain embodiments, a presently disclosed CXCR4 antagonist is administered in accordance with the methods disclosed herein in combination with drugs effective in infectious diseases, such as antibiotics, antibacterial agents or antiviral compounds, *e.g.*, anti HPV agent, anti HIV agent. In particular, the compounds of formula I may be administered together with Maraviroc (UK 427857) from Pfizer; Vicriviroc (SCH-417690, SCH-D), GSK (Ph 11b), GSK's 873140 (also known as AK 602 or ONO 4128) from Schering-Plough's, TAK-652 from Takeda. The compounds of formula I may also be administered together with P-lactams *e.g*., penicillins; cephalosporins; carbapenems; ketolides; quinolones, *e.g*., fluoroquinolones; macrolides, *e.g*, clarithromycin, azithromycin or vancomycin; rifamycins; monobactams; isoniazid; licosamides; mupirocin; sulfonamides; phenicols; fosfomycin; glycopeptides; tetracyclines; streptogramins; chloramphenicol; and oxazolidinone, famciclovir or penciclovir.

The term "anti-viral agent" as used herein includes, but is not limited to, anti-retroviral agent; antibody against virus; *e.g*., anti-HIV antibody; inhibitor of reverse transcriptase; *e.g.*, inhibitor of HIV reverse transcriptase, especially nucleoside analogues, such as Retrovir® (3'-azido-3'-deoxypyrimidine, Zidovudine) and 3'-azido-3'-deoxythymidine (AZT) from GlaxoSmithKline, HIVID® (2',3'-dideoxycytidine, Zalcitabine) from Hoffmann-LaRoche, Videx® or VidexEC® (2',3'dideoxyinosine, Didanosine) from Bristol-Myers-Squibb, Epivir® (Lamivudine) from GlaxoSmithKline, Zerit® (stavudine) from Bristol Myers-Squibb, Viread® (tenofovir DF) from Gilead, Ziagen® (abacavir) from GlaxoSmithKline, Emtriva® (Emtricitabine, FTC) from Gilead Sciences; or non-nucleoside analogues, such as, *e.g*, Rescriptor® (delavirdine) from Pfizer, Sustiva® (Efavirenz) from Bristol Meyer Squibb, Viramune® (nevirapine) from Boehringer-Ingelheim; 11-cyclopropyl-5,11-dihydro-d-methyl-^H^dipyridoP^-b^'^'-eJ-fl^di-azepin-bone, trisodium phosphonoformate, ammonium-21-tungstenato-9-antimonate, 1-.beta.-D-ribofuranoxyl,2,4-triazole-3-carboxamide; inhibitor of viral or retroviral protease, *e.g.,* inhibitor of viral aspartate protease, *e.g.,* inhibitor of HIV protease, such as Aganerase® (amprenavir) fromGlaxoSmithKline, Reyataz® (atazanavir) from Bristol-Myers Squibb, Lexiva® (fosamprenavir) from GSK, Crixivan (Indinavir) from Merck & Co.; Viracept® (nelfmavir) from Agouron, Norvir® (Ritonavir) from Abbott; Fortovase® and Invirase® (saquinavir) from Hoffimann-LaRoche; and other compounds such as lasinavir (5(S)-(tert-butoxycarbonylamino)-4(S)-hydroxy-6-phenyl-2(R)(2,3,4-trimeth-oxyphenylmethyl)-hexanoyl(L)-valyl-N-(2-metoxy-ethyl)-amide), Adriamycin, KVX-478 from Glaxo Wellcome; VX-478 from Vertex; 141W94 from Kissei Pharmaceuticals; AG-1343 from Agouron; KNI-272 from Nippon Mining; U-96988 from Upjohn; BILA-2011 BS (Palinavir) from Boehringer-Ingelheim; compounds preventing virus penetration, such as, *e.g*., polymannoacetate; fusion inhibitors, such as, *e.g.,* Fuzeon® (enfuvirtide, T20) from Hofffmann-LaRoche; or any combination thereof, such as Epzicom® (Abacavir and Lamivudine) from GlaxoKlineSmith, Trizivir® (Abacavir, Lamivudine and Zidovudine) from GlaxoKlineSmith, Truvada® (Emtricitabine and Tenofir DF) from Gilead Sciences, Combivir® (Lamivudine and Zidovudine) from GlaxoKlineSmith, Kaletra® (lopinavir and ritohavir) from Abbott. The term "anti-viral agent" further includes agent which treats the opportunistic infectious which are caused by the immunosuppression resulting from viral infection, *e.g*., HIV infection.

The term "HIV" as used herein includes HIV-1 and HIV-2.

In certain embodiments, a presently disclosed CXCR4 antagonist is administered in accordance with the methods disclosed herein in combination with an anti-retroviral agent or drugs effective in immunosuppressive or immunomodulating regimens, *e.g*., for the treatment or prevention of alio- or xenograft acute or chronic rejection. For example, the compounds of formula I may be used in combination with a JAK3 inhibitor *{e.g.,* CP-690,550), a calcineurin inhibitor, *e.g*., cyclosporin A or FK 506; an mTOR inhibitor, *e.g,* rapamycin, 40-0-(2-hydroxyethyl)-rapamycin, CC1779, ABT578, AP23573, AP23464, AP23675, AP23841 or TAFA-93; an ascomycin having immunosuppressive properties, *e.g*., ABT-281, ASM981, corticosteroids; cyclophosphamide; azathioprine; methotrexate; leflunomide; mizoribine; mycophenolic acid; mycophenolate mofetil; 15-deoxyspergualine or an immunosuppressive homologue, analogue or derivative thereof; a sphingosine-1-phosphate receptor agonist, *e.g*., FTY720; monoclonal antibodies to leukocyte receptors, e.g., MHC, CD 2, CD3, CD4, CD7, CDS, CD11a/CD18, CD25, CD27, CD28, CD40. CD45, CD58, CD80, CD86, CD137, ICOS, CD 150 (SLAM), 0X40, 4-1 BB or to their ligands, *e.g*., CD 154, or antagonists thereof; other immunomodulatory compounds, *e.g*., *a* recombinant binding molecule having at least a portion of the extracellular domain of CTLA4 or a mutant thereof, *e.g.,* an at least extracellular portion of CTLA4 or a mutant thereof joined to a non-CTLA4 protein sequence, *e.g.,* CTLA4Ig *(e.g.,* designated ATCC 68629) or a mutant thereof, *e.g.,* LEA29Y; adhesion molecule inhibitors, *e.g.,* LFA-1 antagonists, ICAM-1 or -3 antagonists, VC AM-4 antagonists or VLA-4 antagonists; or antichemokine antibodies or antichemokine receptor antibodies or low molecular weight

In certain embodiments, a presently disclosed CXCR4 antagonist is administered in accordance with the methods disclosed herein in combination with COX-2 inhibitors, mTOR inhibitors, gonadorelin agonists, and anti-androgens.

The term "COX-2 inhibitors" relates to compounds which inhibit the cyclooxygenase type 2 enyzme (COX-2) and which possess antiproliferative activity such as celecoxib (Celebrex®), rofecoxib (Vioxx®) and lumiracoxib (COX189).

The term "mTOR inhibitors" relates to compounds which inhibit the mammalian target of rapamycin (mTOR) and which possess antiproliferative activity such as sirolimus (Rapamune®), everolimus (CerticanF), CCI-779 and ABT578.

The term "gonadorelin agonist" as used herein includes abarelix, goserelin and goserelin acetate. Goserelin is disclosed in U.S. Patent No. 4,100,274 and can be administered, *e.g.,* in the form as it is marketed, *e.g.,* under the trademark ZOLADEX™.

The term "anti-androgens" as used herein includes bicalutamide (CASODEXT™), which can be formulated, *e.g.,* as disclosed in U.S. Patent No. 4,636,505.

The structure of the active agents identified by code nos., generic or trade names may be taken from the actual edition of the standard compendium "The Merck Index" or from databases, *e.g..* Patents International *(e.g.,* IMS World Publications). The above-mentioned compounds, which can be used in combination with a compound of formula I, can be prepared and administered as described in the art such as in the documents cited above.

Where the compounds of formula I are administered in conjunction with gammaglobulin, immunoglobulin, cytokine, anti-inflammatory agent, anti-infective agent, e.g., anti-viral agent or antibiotic, or another CXCR4 antagonist, dosages of the co-administered compound will of course vary depending on the type of co-drug employed, on the specific drug employed, on the severity of the condition being treated and so forth.

The invention is further illustrated in the following example.

### EXAMPLES

### Example 1: Clinical Trial Results-Plerixafor is a potential therapy for myelokathexis, WHIM syndrome.

Study Design: This was an open label, single Center, phase I study to examine the hematological effects, pharmacokinetics and safety of plerixafor in patients with myelokathexis attributable to mutations of CXCR4, utilizing serial, escalating doses of plerixafor administered on days 1,3,5, 8, and 10. Five intrapatient escalating dose levels, 20 mcg/kg, 40 mcg/kg, 80 mcg/kg, and 240 mcg/kg were exam in d. The subjects were patients at the University of Washington General Clinical Research Center for up to 10 days; the study requires the subject to be available for up to 14 days. Patients were monitored for hematological effects of plerixafor and observed for adverse effects. If a normal blood neutrophil count is achieved and maintained for at least 24 hours prior to the highest dose, dosing of plerixafor was discontinued.

| | |
|---|---|
| Ages Eligible for Study: | 18 Years and older |
| Genders Eligible for Study: Both Accepts Healthy Volunteers: No | |

### Criteria

### Inclusion Criteria:

- age over 18 years,
- WBC less than 3.0 x 10⁹/L,
- Absolute neutrophil count less than 2.0 x 10⁹/L,
- platelets greater than 100 x 10⁶/L,
- creatinine less than 2.0/mg /dl,
   Creatinine clearance > 60 ml/min calculated, SCOT, SGPT,
- bilirubin < 2. 5 upper limit of normal,
- ECOG status 0 or 1,
- mutation in CXCR4 identified,
- on no G-CSF, GM-CSF for > 3 weeks, p
- patient signs consent,
- accepts contraception

### Exclusion Criteria :

- <18 years old,
- sensitivity to plerixafor,
- pregnant,
- prisoner,
- decisionally impaired,
- judged unlikely to comply,
- illness that may interfere with interpretation of results,
- leukemia,
- malignancy,
- active infection requiring antibiotics within one week of study drug administration,
- history of cardiac conduction or EKG abnormality,
- previous experimental therapy within one week.

Results and Discussion:: We enrolled 6 patients (4 female, 2 male, ages 28 to 73 years) in this dose escalation study, with informed consent and investigational review board approval of the University of Washington and Federal approval for investigational use of plerixafor. Five patients from three different families had the same mutation (R334ter); the other patient had a novel mutation (S324fs365ter). Single subcutaneous doses of plerixafor, increasing from 0.02 to 0.24 mg/kg (0.02 mg/kg, 0.04 mg/kg, 0.08 mg/kg, 0.16 mg/kg, 0.24 mg/kg) were administered at 2 to 4 day intervals. Complete blood counts were determined at 1, 3, 6, 9 and 24 hours with an automated counter and leukocyte differential counts confirmed manually. CD34⁺ cells and lymphocyte subtypes were measured by FACS before and 6 hours after the 0.08 mg/kg dose. Plerixafor was discontinued if neutrophils were >2.0 x 10⁹/L at 24 hours, if serious adverse events or illness occurred, or after testing the 0.24 mg/kg dose. Outcome measures were the patients' complete blood cell counts, CD34⁺ cell counts and lymphocyte subtypes compared with five normal subjects similarly treated with plerixafor. Results were compared with five similarly studied normal subjects utilizing Student's t test for comparisons of means of normal subjects and controls and the ratio paired t test for comparison of baselines and responses for each category of leukocytes.

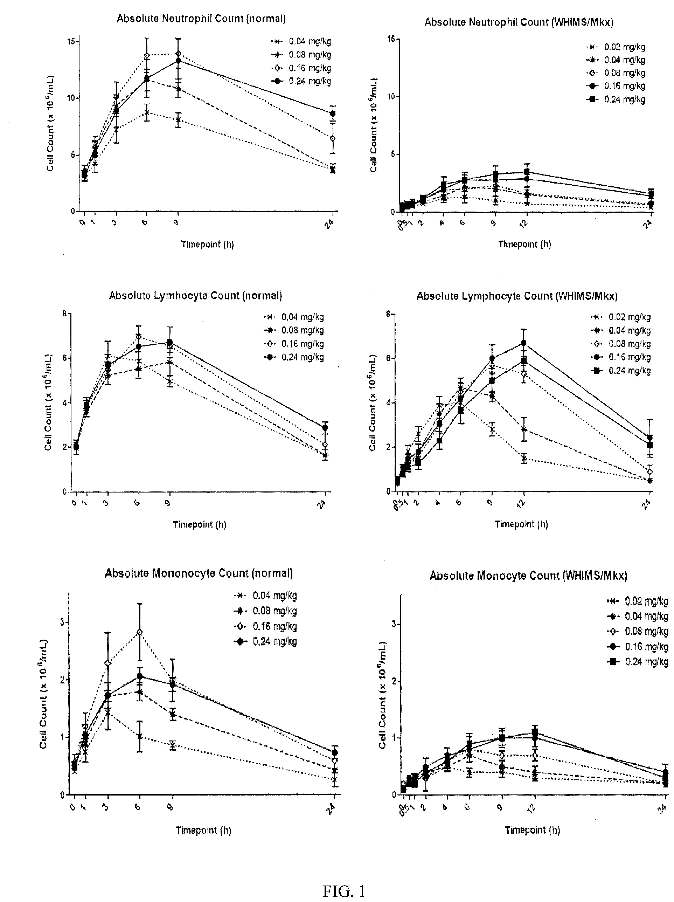

With reference to Fig. 1, all 6 patients showed prompt leukocytosis with maximum blood neutrophils and lymphocytes at 6 to 12 hours, declining toward baseline by 24 hours. One patient had neutrophils greater than 2.0 x 10⁹/L 24 hours after the 0.16 mg/kg dose. Having reached this target for the study, he was not given the final 0.24 mg/kg dose. Another patient achieved 2.0 x 10⁹/L 24 hours after the 0.24 mg/kg dose. One patient discontinued the trial after the 0.08 mg/kg dose when she had a recurrence of pneumonia. In the patient group, neutrophils peaked at 4.5±0.78 x 10⁹/L (group mean of highest observed values; range: 1.8 to 7.3 x 10⁹/L). The absolute increase after single doses of plerixafor was less than for normal subjects, but the relative increase was greater. Comparing peak neutrophil responses, there was a 4.4 fold increase for the controls and an 8.2 fold increase for the patients. For all patients, neutrophils had returned to baseline before the next dose of plerixafor was administered. Patients' lymphocyte responses were proportionally greater than their neutrophil responses. Absolute lymphocyte counts transiently reached normal levels in the patients, increasing approximately 14-fold from baseline to the peak levels.

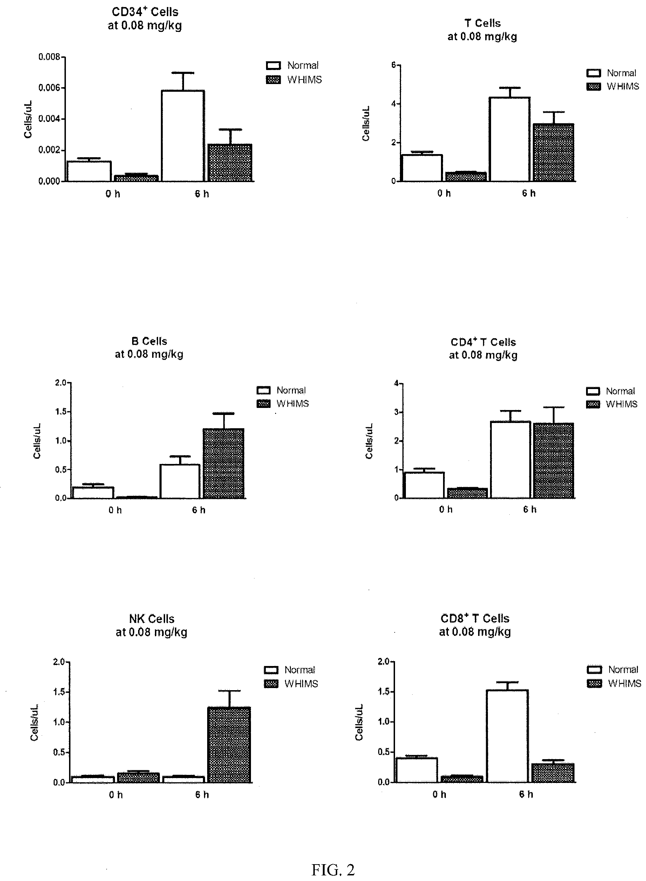

With reference to Fig. 2, all categories of lymphocytes tested increased in patients after plerixafor, and some responses were greater than for the controls. The absolute levels of B cells showed the largest increase over baseline, increasing approximately 40-fold. Patients' CD34⁺ cells increased almost 6-fold at the 0.08 mg/kg dose. Hematocrit, hemoglobin and platelet counts were stable through the 10 day testing period, except for one patient with severe iron deficiency anemia who responded well to oral iron initiated during the study. None of the patients experienced significant adverse effects

This trial demonstrates that subcutaneous administration of single doses of plerixafor can transiently correct neutropenia and lymphocytopenia in patients with myelokathexis/WHIM syndrome. The patients' responses were qualitatively similar to those of normal controls. Although the quantitative increase in neutrophils was less than in normal controls, the proportional increase was greater. This suggests that the mechanisms for release of neutrophils from the mature marrow storage pool are intact, but the "hyper-mature" and apoptotic neutrophils in the marrow have lost capacity to circulate normally. Based on these results, we believe that regular, perhaps daily, plerixafor administration may have a greater ability to increase and maintain blood neutrophil levels.

Lymphocyte counts increased much more dramatically, and all lymphocyte subtypes increased in the patients. Lymphocytes have far more complex circulatory patterns than neutrophils, entering and leaving the blood in the marrow, spleen and other tissues. Based on these results, we believe that lymphocyte mobilization from the tissues to the blood and apparently correcting the trafficking of lymphocytes may be very important for these patients. As noted above, patients with WHIM syndrome typically have hypogammaglobulinemia, and the pattern of their infections, particularly their propensity to develop severe problems with warts, appears to be the result of a selective immunodeficiency which might be corrected with chronic plerixafor therapy.

All patients showed prompt leukocytosis with maximum blood neutrophils and lymphocytes at 6 to 12 hours. Blood neutrophils peaked at 6 to 12 hours, increasing from a mean baseline of 0.4±0.1 x 10⁹/L, to mean peak of 4.5±0.78 x 10⁹/L, Lymphocytes also increased; the greatest increase was in B cells (CD 19+ cells), a greater than 40-fold increase over baseline at the 0.08 mg/kg dose. None of the patients experienced any significant adverse effects.

Based on these results, we established that plerixafor is a promising treatment for WHIM syndrome.

## Claims

1. A compound for use in a method of:
- treating or preventing WHIM syndrome in a human;
- treating or preventing myelokathexis in a human; or
- correcting neutropenia and/or lymphocytopenia in a human patient with myelokathexis or WHIM syndrome;
wherein the compound is a CXCR4 antagonist that is 1,1'-[1,4-phenylene-bis-(methylene)]-bis-1,4,8,11-tetraazacyclotetradecane or a pharmaceutically acceptable salt or metal complex thereof.

2. A compound for use according to claim 1 wherein the method comprises testing for the presence of a CXCR4 mutation in a human having myelokathexis or WHIM syndrome;
selecting a human having the CXCR4 mutation; and
administering to the selected human a CXCR4 antagonist as defined in claim 1 in an amount effective to treat or prevent myelokathexis or WHIM syndrome.

3. A compound for use according to claim 1, wherein the compound is in the form of an acid addition salt.

4. A compound for use according to claim 3, wherein the acid addition salt is hydrochloride.

5. A compound for use according to claim 1 or 2, wherein the human has the CXCR4 mutation R334.

6. A compound for use according to claim 1 or 2, wherein the human has the CXCR4 mutation S324fs365.

7. A compound for use according to claim 1, wherein the compound is administered at a dose range of between about 0.04 mg/kg to 0.24 mg/kg.

8. A compound for use according to claim 1, wherein the compound is administered to the human by a subcutaneous route.

9. A compound for use according to claim 1, wherein the compound is administered in combination with G-CSF.

## Patentansprüche

1. Verbindung zur Verwendung in einem Verfahren von Folgendem:
- Behandeln oder Vorbeugen des WHIM-Syndroms beim Menschen;
- Behandeln oder Vorbeugen der Myelokathexis beim Menschen; oder
- Korrigieren von Neutropenie und/oder Lymphozytopenie bei einem menschlichen Patienten mit Myelokathexis oder WHIM-Syndrom;
wobei die Verbindung ein CXCR4-Antagonist ist, der 1,1'-[1,4-Phenylen-bis(methylen)]-bis-1,4,8,11-tetraazacyclotetradecan oder ein pharmazeutisch verträglicher Salz- oder Metallkomplex davon ist.

2. Verbindung zur Verwendung nach Anspruch 1, wobei das Verfahren Folgendes umfasst:
Testen auf das Vorhandensein einer CXCR4-Mutation bei einem Menschen mit Myelokathexis oder WHIM-Syndrom;
Auswählen eines Menschen mit der CXCR4-Mutation; und
Verabreichen an den ausgewählten Menschen eines CXCR4-Antagonisten nach Anspruch 1 in einer Menge, die zur Behandlung oder Vorbeugung von Myelokathexis oder WHIM-Syndrom wirksam ist.

3. Verbindung zur Verwendung nach Anspruch 1, wobei die Verbindung in Form eines Säureadditionssalzes vorliegt.

4. Verbindung zur Verwendung nach Anspruch 3, wobei das Säureadditionssalz Hydrochlorid ist.

5. Verbindung zur Verwendung nach Anspruch 1 oder 2, wobei der Mensch die CXCR4-Mutation R334 aufweist.

6. Verbindung zur Verwendung nach Anspruch 1 oder 2, wobei der Mensch die CXCR4-Mutation S324fs365 aufweist.

7. Verbindung zur Verwendung nach Anspruch 1, wobei die Verbindung in einem Dosierungsbereich zwischen etwa 0,04 mg/kg bis 0,24 mg/kg verabreicht wird.

8. Verbindung zur Verwendung nach Anspruch 1, wobei die Verbindung dem Menschen auf einem subkutanen Weg verabreicht wird.

9. Verbindung zur Verwendung nach Anspruch 1, wobei die Verbindung in Kombination mit G-CSF verabreicht wird.

## Revendications

1. Composé pour une utilisation dans un procédé de :
- traitement ou prévention du syndrome WHIM chez un être humain ;
- traitement ou prévention de la myélokathexis chez un être humain ; ou
- correction de la neutropénie et/ou de la lymphocytopénie chez un patient humain atteint de myélokathexis ou du syndrome WHIM ;
dans lequel le composé est un antagoniste de CXCR4 qui est le 1,1'-[1,4-phénylène-bis-(méthylène)]-bis-1,4,8,11-tétraazacyclotétradécane ou un sel ou complexe métallique pharmaceutiquement acceptable de celui-ci.

2. Composé pour une utilisation selon la revendication 1, dans laquelle le procédé comprend un test de la présence d'une mutation de CXCR4 chez un être humain atteint de myélokathexis ou du syndrome WHIM ;
la sélection d'un être humain présentant la mutation de CXCR4 ; et
l'administration à l'être humain sélectionné d'un antagoniste de CXCR4 tel que défini dans la revendication 1 dans une quantité efficace pour traiter ou prévenir la myélokathexis ou le syndrome WHIM.

3. Composé pour une utilisation selon la revendication 1, dans laquelle le composé se présente sous la forme d'un sel d'addition d'acide.

4. Composé pour une utilisation selon la revendication 3, dans laquelle le sel d'addition d'acide est le chlorhydrate.

5. Composé pour une utilisation selon la revendication 1 ou 2, dans laquelle l'être humain présente la mutation R334 de CXCR4.

6. Composé pour une utilisation selon la revendication 1 ou 2, dans laquelle l'être humain présente la mutation S324fs365 de CXCR4.

7. Composé pour une utilisation selon la revendication 1, dans laquelle le composé est administré à une plage de doses comprise entre environ 0,04 mg/kg à 0,24 mg/kg.

8. Composé pour une utilisation selon la revendication 1, dans laquelle le composé est administré à un être humain par voie sous-cutanée.

9. Composé pour une utilisation selon la revendication 1, dans laquelle le composé est administré en combinaison avec G-CSF.
